# EUROPEAN PATENT SPECIFICATION

(11) **EP 2 210 589 B1**
(45) Date of publication and mention of the grant of the patent: **06.05.2015**
(21) Application number: 09151127.9
(22) Date of filing: 22.01.2009
(51) Int. Cl.: A61K 9/127, A61K 38/04, A61K 38/16

(54) **Vesicular phospholipid gels comprising proteinaceous substances**
Vesikuläre Phospholipid-Gele mit proteinösen Substanzen
Gels phospholipidiques vésiculaires comportant des substances protéiques

(43) Date of publication of application: 28.07.2010
(60) Divisional application: 13155618.5
(73) Proprietor: Ludwig-Maximilians-Universität München, 80539 München (DE)
(72) Inventor: Winter, Gerhard, 82377 Penzberg (DE); Brandl, Martin, DK- 5000 Odense C (DK); Schulze, Sandra, 81543 München (DE); Tian, Weiwei, PRC- 300200 Tianjin (CN)
(74) Representative: Neuefeind, Regina

(56) References cited:
- EP-A- 1 674 081
- WO-A-98/03641
- WO-A-2007/043057
- DE-A1- 4 430 592
- DE-A1- 19 940 227
- US-A- 5 711 965

## Description

### FIELD OF THE INVENTION

The present invention relates to a pharmaceutical composition for sustained release of a pharmaceutically active compound, the composition comprising a vesicular phospholipid gel. More particularly, the invention relates to a pharmaceutical composition comprising at least one proteinaceous substance as the pharmaceutically active compound in encapsulated form, the at least one proteinaceous substance being a biologically active protein, peptide or polypeptide. Furthermore, the present invention relates to a method for the production of said pharmaceutical composition comprising dual asymmetric centrifugation and to the use of said pharmaceutical composition for immunotherapy and/or for stimulating selective tissue regeneration in the treatment of surgical defects in the course of surgical interventions.

### BACKGROUND

Many promising new drugs are of peptide or protein origin, opening new dimensions in the treatment of diseases. The higher selectivity of proteins, peptides or polypeptides, or the like often allows a better treatment of serious, life-threatening, and chronic diseases such as cancer, rheumatoid arthritis, hepatitis and others. However, the main challenge in the use of proteinaceous substances as therapeutically active compounds is to overcome their low bioavailability and instability. Due to their fragile, three-dimensional macromolecular structure, proteinaceous substances are often susceptible to a variety of chemical or physical degradation pathways. Such biologically active compounds can thus only be formulated with difficulty into pharmaceutical compositions which are sufficiently stable on storage and have an adequate bioavailability.

Proteinaceous substances susceptible to degradation in the gastrointestinal tract have to be supplied as parenteral or topical preparations which exhibit immediate release or high release rates within a short time in order to obtain adequate plasma levels and high levels of drug concentration at the local site of action. In many cases, repeated administrations are required to achieve the expected therapeutic effect for an extended period of time. Long-term sustained delivery of such pharmaceutically active compounds is thus essential for the practical use of these medications. Another problem associated with frequent administration of such pharmaceutically active compounds is patient compliance. It is often difficult to get a patient to follow a prescribed dosage regimen, particularly when the prescription is for a chronic disorder and the respective pharmaceutical active compound has side effects.

Hence, it would be very desirable to provide a system for the delivery of such proteinaceous substances at a sustained release rate over a long period of time. Such a sustained release system would have the advantage of optimizing the therapeutic efficacy, minimizing side-effects and toxicity, and enhancing patient compliance.

Sustained or slow release compositions are designed to contain higher concentrations of the medicament and are prepared in such a manner as to effect sustained or slow release over an extended period of time. Such slow release therapeutic drug dosage forms have inherent advantages over conventional, immediate release dosage forms. The advantages include less frequent dosing of the pharmaceutically active compound and resultant patient regime compliance, a more sustained drug blood level response or a more sustained drug level at the local site of action, the possibility of effecting therapeutic action with a lower drug dosage and the mitigation of side effects. By providing a slow and steady release of the pharmaceutically active compound over time, absorbed drug concentration spikes are mitigated or eliminated by effecting a smoother and more sustained blood or tissue level response.

For this purpose a retard formulation has to meet some criteria, namely providing a uniform and constant liberation of the drug and being effective for an extended period of time. It is also important that such a formulation can easily and cost efficient produced by a reproducible manufacturing process applicable for a number of different biologically active agents. Various methods have been used and proposed for the sustained release of small molecule drugs. Such methods include slow-release, orally administered compositions, such as coated tablets, formulations designed for gradual absorption, such as transdermal patches, and slow-release microparticles, and implants such as "rods" implanted under the skin.

One method by which the gradual release of a bioactive agent has been proposed is a so-called "depot" injection. In this method, a bioactive agent is formulated with carriers providing a gradual release of active agent over a period of a number of hours or days. These are often based upon a degrading matrix which gradually disperses in the body to release the active agent. The most common of the established methods of depot injection relies upon a polymeric depot system. This is typically a biodegradable polymer such as poly (lactic acid) (PLA) and/or poly (lactic-co-glycolic acid) (PLGA). The polymers entrap the active agent and are gradually degraded releasing the agent by slow diffusion and/or as the matrix is absorbed. Examples of such systems include those described in U.S. Pat. No. 4,938,763, U.S. Pat. No. 5,480,656 and U.S. Pat. No. 6,113,943 and can result in delivery of active agents over a period of up to several months. These systems do, however, have a number of limitations including the complexity of manufacturing.

So far a broad variety of parenteral depot systems was investigated and described in the prior art. However, at the moment no controlled release system for the delivery of proteins is on the market. This limited success can be explained by the fragile three-dimensional macromolecular structure of proteins which makes them susceptible to a variety of chemical and physical degradation pathways during manufacturing, storage, and release [K. Fu, A. M. Klibanov, and R. Langer, Protein stability in controlled-release systems, Nature Biotechnology. 18 (2000) 24-25.; C. Perez, I.J. Castellanos, H.R. Costantino, W. Al-Azzam, K. Griebenow, Recent trends in stabilizing protein structure upon encapsulation and release from bioerodible polymers, Journal of Pharmacy and Pharmacolog. 54 (2002) 301-313.; L. Jorgensen, E.H. Moeller, M. van de Weert, H.M. Nielsen, and S. Frokjaer, S., Preparing and evaluating delivery systems for proteins, European Journal of Pharmaceutical Sciences. 29 (2006) 174-182.; M. van de Weert, J.A. Hering, P.I. Haris, Fourier transform infrared spectroscopy, Biotechnology: Pharmaceutical Aspects. 3 (2005) 131-166.]. In particular, the frequently-used synthetic biodegradable polymers PLA and PLGA have several shortcomings. During the release period, for instance, the polymer matrix undergoes bulk erosion and reactive degradation products are trapped within the system. Consequently, the incorporated proteins are faced with a completely altered microenvironment. In particular the significant pH drop [K. Fu, D.W. Pack, A.M. Klibanov, R. Langer, Visual evidence of acidic environment within degrading poly(lactic-co-glycolic acid) (PLGA) microspheres, Pharmaceutical research. 17 (2000) 100-106], the increase of the osmotic pressure [A. Lucke, J. Kiermaier, A. Goepferich, Peptide acylation by poly(a-hydroxy esters), Pharmaceutical research. 19 (2002) 175-181] and the accumulation of reactive species [A. Brunner, K. Mader, A. Goepferich, pH and osmotic pressure inside biodegradable microspheres during erosion, Pharmaceutical research. 16 (1999) 847-853] have been identified as causes for protein unfolding, aggregation, and chemical degradation.

As the restrictions of polymer based depot systems became obvious, another group of sustained release systems based on lipid materials was investigated. Lipids are excellently biocompatible, what has been shown in animal studies. Due to their natural origin, they are non-toxic and degradable into fatty acids, glycerol, alcohols etc. and finally metabolized by the physiological metabolic cycles of mammals. Furthermore, lipids are by definition rather hydrophobic and the diffusion of water into lipid systems is generally low. In contrast to polymer systems (like PLGA) lipid systems therefore show different swelling, degradation and release behaviour, which is particularly advantageous for protein drugs and their release.

Liposomes, also known as lipid vesicles, are completely closed lipid bilayer membranes which contain an entrapped volume comprising an aqueous medium. The lipid bilayer is often composed of phospholipids such as lecithin and related materials such as glycolipids. Liposomes may be unilamellar, having a single membrane bilayer, or multilamellar, having more than one membrane bilayer and having an aqueous space between different membrane bilayers. The bilayer is composed of two lipid monolayers, each having a hydrophobic portion that is oriented towards each other with the hydrophilic portion facing outwards towards the aqueous phases. Liposomes are formed when phospholipids or other suitable amphipathic molecules are allowed to swell in water or aqueous solution. If water-soluble materials are included in the aqueous phase during this process, the material may become trapped in the aqueous phase between the lipid bilayers. Similarly, lipophilic materials may be dissolved in the lipid and be incorporated into the bilayers themselves, although if the lipophilic material also has a polar group, this group may extend into the inner or outer aqueous phase. However, one of the main restrictions of such systems is their low encapsulation efficiency of water soluble drugs such as peptides and proteins. Due to the distribution of the drug in the aqueous phase inside and outside of the liposomal vesicles most manufacturing processes resulted in yields below 50% (Immunomethods 4, 201-209, 1994). Liposomes are known as means for controlled release and targeting of pharmaceutical agents (for example, see the overview in Ullrich, Biosci Rep. 2002; 22(2): 129-50). However, although injectable liposome preparations appear beneficial over other alternatives, the use of such systems according to the state of the art leaves still important drawbacks and unresolved problems open (low storage stability due to the leakage of the active drug out of the vesicles, low encapsulation yields, and a very short depot effect).

For the manufacturing of substance-loaded liposomes various processes have been reviewed by Lasch *et al*. (J. Lasch et al., Preparation of Liposomes; in "Liposomes - a practical approach", V.P. Torchilin and V. Weissing, Ed., 2nd edition (2003)). However, in most cases it is desirable to provide liposomes which are uniform with respect to the lamellarity and size.

Phospholipids when dispersed and sheared in aqueous medium at high lipid contents result in highly viscous dispersions up to semisolid consistency, so called "vesicular phospholipid gels" (VPGs). VPGs, as conventional liposomes, contain both aqueous compartments and bilayer-membranes suited to accommodate water-soluble and lipid-soluble drugs, respectively. Their potential as depot formulations for sustained release of small molecule drugs was investigated (M. Brandl et al., Proc. International Symposium on Controlled Release of Bioactive Materials 1995, 22, 472-473) and different approaches have been described to load VPGs with small molecule drugs (reviewed by M. Brandl, J. of Liposome Research 2007, 17, 15-26).

The choice of the proper technology largely depends on the physicochemical characteristics of the drug, when manufacturing conventional liposomes. According to the drugs's ability to interact with phospholipid bilayers or not, the substances may be divided into two main categories: Water soluble substances (which upon loading into liposomes are truly entrapped or encapsulated within the aqueous compartments of liposomes) and amphiphilic or lipophilic substances which are incorporated into or associated with the bilayers. In the case of VPGs, the physicochemical properties of the drug is of much less importance for the quality and characteristics of the preparation.

The lamellarity of said liposomes may be reduced by using commonly known processes such as high-pressure homogenization, extrusion, the freeze-thaw method or ultrasonification. Industrial scale production of liposomes mainly uses high-pressure homogenizers and filters extruders. For instance, WO 96/05808 A1 proposes a liposomal preparation suitable for use in pharmaceutics and medicine, in particular as a depot for small molecule drugs as encapsulated substances. The preparation is produced by mixing a lipid or lipids, water and the active substance to be incorporated, and subsequently removing the solvents by evaporation and dispersal in water, followed by single or multiple high-pressure homogenization (at the most 50 times) at a pressure between 50 and 1600 bar (5-160 MPa) and, optionally, freeze/thaw treatment. However, what this and most of the further liposome preparation techniques described in the art have in common is that an outstanding technological know-how is required, not at least to limit the danger for the environment resulting from the use of hazardous organic solvents, that the equipment required for some of these processes is expensive, bulky and unacceptable for many laboratories. Beside these environmental and financial concerns the harsh manufacturing process itself inhere several sources for drug destabilisation during preparation, which is in particular critical for expensive and sensitive substances such as biologically active proteins, peptides and polypeptides. Hence, there is a strong need in the technical field to develop other advantageous lipid based delivery systems carrying proteinaceous substances as the pharmaceutically active compounds in encapsulated form.

WO 2006/069985 A2 relates to a method for producing lipid-based nanoparticles using a dual asymmetrical centrifuge. A "dual asymmetric centrifuge" is a centrifuge where in addition to the processes of classic centrifugation the vessel containing the material to be centrifuged is rotated on a second rotation axis in addition to the first direction of rotation. This results in constant mixing of the material introduced into the dual asymmetrical centrifuge. However, in order to facilitate homogenization of the material to be centrifuged and to standardize the particle size, homogenization aids such as glass beads are arranged within the vessel containing the material. Hence, this method has the drawback that a large percentage of the expensive and valuable biological substances to be encapsulated may stick to said glass beads or homogenization aids.

WO 98/03641 A1 describes compositions and methods for the transdermal transport of molecules, including macromolecules. Further described are compositions and methods for the transdermal transport of molecules to treat conditions in a patient.

DE 199 40 227 A1 describes a phospholipid gel which is stabilized against liquefaction by addition of an alcohol or a sugar. This gel may be used for the production of cosmetic or pharmaceutical formulations.

US 5,711,965 A discloses an alcoholic, aqueous gel-like phospholipid composition which contains, as alcohols, ethanol, 1-propanol or 2-propanol, and is characterized in that this composition is a liposomal gel composed of 15 to 30 parts by weight of a phospholipid concentrate, 14 to 20 parts by weight of alcohol and 50 to 71 parts by weight of an aqueous solution as the remainder.

DE 44 30 592 A1 describes a liposomal vesicular preparation for encapsulation of active ingredients which is based on homogeneous unilamellar liposomal vesicular lipids, has a vesicular structure and forms carriers of active ingredients. The liposomal vesicular preparation is described to be a semi-solid liposomal vesicular lipid gel. The membrane forming amphiphiles are members from the group consisting of lipids, phospholipids and synthetic amphiphiles.

Furthermore, from a drug delivery point of view, many sustained release compositions in the art not only have the disadvantage of accepting only relatively low drug loads, but also having a "burst/lag" release profile. Such sustained release compositions often show an initial burst of drug release when the respective composition is administered. This is followed by a period of low release, while the degradation of the matrix of the depot begins, eventually followed by a final increase in the release rate (biphasic or triphasic release kinetics). Evidently, from a functional and toxicological point of view this burst release is undesirable and could be dangerous. It may also limit the equilibrium concentration which can be provided due to the danger of adverse effects at the "peak" point. Hence, there is a strong need in the technical field to provide alternative means for long-term sustained and constant delivery of biologically active proteins, peptides and polypeptides which do not have an undesired initial burst release.

Accordingly, it is an object of the present invention to provide such improved pharmaceutical compositions for sustained release of a pharmaceutically active compound, the compositions comprising at least one proteinaceous substance as the pharmaceutically active compound in encapsulated form. In summary, based on the above presented technical field and the resulting needs for an improved depot system the following objectives were set:
- Little or no aggregation and disintegration of biological substances occurring during manufacturing and storage of the system
- Little or no aggregation and disintegration of biological substances during release (release of the monomeric protein)
- Sustained and continuous release, preferably close to zero order release kinetics, with an acceptable low initial burst
- Easy, cheap (including the used exciepients), reproducible, fast and scalable manufacturing process
- Possibility to process high drug loads
- Excellent biocompatibility and safety of the system
- High effectiveness and wide applicability

These and other objectives of the present invention that will become apparent from the description are accomplished by the pharmaceutical composition and by the method for the production of said pharmaceutical composition according to the independent claims. Preferred embodiments are defined by the dependent claims.

### SUMMARY OF THE INVENTION

In a first aspect, the present invention relates to a pharmaceutical composition for sustained release of a pharmaceutically active compound, comprising a vesicular phospholipid gel having a viscosity of at least 3.0 Pa·s at 25°C at a shear rate of 38 s⁻¹ comprising tightly packed liposomes having a vesicular structure, wherein
(i) the vesicular phospholipid gel comprises at least one proteinaceous substance as the pharmaceutically active compound in encapsulated form, the at least one proteinaceous substance being a biologically active protein, peptide or polypeptide, wherein the at least one proteinaceous substance has a molecular weight of at least 3,400 g/mol; and
(ii) the gel exhibits a substantially continuous release rate of the at least one proteinaceous substance over a period of at least 200 hours when exposed at about 37°C to an aqueous medium.

In preferred embodiments of the invention, the phospholipid is selected from the group consisting of phosphatidylcholines, phosphatidylethanolamines, phosphatidylinosites, phosphatidylserines, cephalines, lysolecithines, phosphatidylglycerols and mixtures of several such phospholipids. Preferably, a blend of phosphatidylcholines and/or phosphatidylglycerols is used as the phospholipid.

In further preferred embodiments of the present invention, the pharmaceutical composition has a phospholipid content of at least 30 wt-%.

In further specific embodiments, the pharmaceutical composition further comprises a lipid selected from the group consisting of fatty acids, monoglycerides, diglycerides, triglycerides, sorbitan fatty acid esters, sphingolipids, cholesterol, waxes, and salts and derivatives thereof.

In preferred embodiments, the weight ratio between the phospholipid and the at least one proteinaceous substance is between 1000:1 1 and 5:1.

Preferably, the proteinaceous substance is an antibody selected from the group consisting of abciximab, adalimumab, alemtuzumab, basiliximab, bevacizumab, cetuximab, daclizumab, eculizumab, efalizumab, ibritumomab tiuxetan, infliximab, muromonab-CD3, natalizumab, omalizumab, palivizumab, panitumumab, ranibizumab, gemtuzumab ozogamicin, rituximab, tositumomab and trastuzumab.

Also preferably, the proteinaceous substance is a growth factor selected from the group consisting of epidermal growth factors, neuregulins, fibroblast growth factors, hematopoietic cytokines, insulin-like growth factors, interleukins, neurotrophic factors, platelet derived growth factors, transforming growth factors, tumor necrosis factors and VEGF.

In further preferred embodiments of the present invention, the proteinaceous substance is a proteohormone selected from the group consisting of cytokines such as granulocyte-colony stimulating factor, interferons and interleukins; hematopoetic hormones such as erythropoietin; and peptide hormones such as follicle stimulating hormone, luteinizing hormone, gonadotropin-releasing hormone, glucagon and insulin.

In other specific embodiments, the pharmaceutical composition exhibits a release of the at least one proteinaceous substance of less than 20% during 24 hrs when exposed at 37°C to an aqueous medium.

In preferred embodiments of the invention, the pharmaceutical composition further comprises at least one excipient which
(i) modifies the release of the at least one proteinaceous substance from the liposomes and/or
(ii) modifies the biodegradation of the vesicular phospholipid gel and/or
(iii) stabilizes the at least one proteinaceous substance and/or
(iv) modifies the solubility of the at least one proteinaceous substance.

In case, the pharmaceutical composition further comprises at least one excipient, the excipient is preferably selected from the group consisting of hydrophilic polymers, sugars, polyols, surfactants, antioxidants, cyclodextrins, water miscible organic solvents and water-soluble salts.

In a second aspect, the present invention relates to a method for the production of such a pharmaceutical composition, comprising
(i) providing a preparation comprising at least one phospholipid and at least one proteinaceous substance, wherein the at least one proteinaceous substance is a biologically active protein, peptide or polypeptide;
(ii) homogenizing the preparation of (i) in a dual asymmetric centrifuge.

In preferred embodiments of the invention, the method further comprises controlling and/or adjusting the temperature of the preparation during the centrifugation process.

In further preferred embodiments of the present invention, homogenizing the preparation is performed in a dual asymmetric centrifuge without using any homogenization aid.

In a further aspect, the present invention relates to the inventive pharmaceutical composition for the use in the continuous systemic or local delivery of a proteinaceous substance by applying the composition to the human or animal body via different administration routes.

In a further aspect, the present invention relates to such a pharmaceutical composition for the use in immunotherapy, comprising administering to a subject in need thereof, a therapeutically effective amount of the pharmaceutical composition, wherein the immunotherapy comprises (i) treating malignant cell growth by stimulating the immune system or (ii) treating an autoimmune disease or preventing rejection of transplanted organs or cells by reducing the normal immune response.

In a further aspect, the present invention relates to such a pharmaceutical composition for the use in stimulating selective tissue regeneration in the treatment of tissue defects, comprising administering to a subject in need thereof, a therapeutically effective amount of the pharmaceutical composition.

In preferred embodiments of the invention, the tissue is selected from the group consisting of joints, muscle tissue, connective tissue, tendons, organs and skin.

In preferred embodiments of the invention, administering the pharmaceutical composition comprises injection, needle-free injection, vaginal application, nasal application, urethral application, topical application, application in the oral cavity (e.g. buccal), application to wounds or during or after surgery.

### BRIEF DESCRIPTION OF THE DRAWINGS

**Figure 1****:**
   **A:** Effect of the phospholipid content on the viscosity and on the gel strength (measured by texture analysis) of VPGs. The VPGs were prepared either with Phospholipon 85G or 90G.
   **B:** Effect of the phospholipid content on the viscosity and on the gel strength (measured by texture analysis) of VPGs. The VPGs were prepared with LIPOID E80 without any drug incorporation.
**Figure 2****:**
   Cumulative release of FITC-Dextran from VPGs with different phospholipid concentrations in tubes (40%, 45%, 50%, 55% and 60%, Phospholipon 85G), FITC-Dextran 2.2 mg/g, centrifugation 60 mins (average±SD, n=3).
**Figure 3****:**
   PCS analysis of redispersed VPGs consisting of 500 mg/g phospholipids (LIPOID E80).
**Figure 4****:**
   **A:** Effect of the preparation process on the stability of EPO. SDS-PAGE with subsequent silver staining of: molecular standard maker (lane 1 and 10); EPO extracted from VPGs by chloroform (lane 2-5), reference EPO (lane 6-9).
   **B:** Effect of the preparation process on the stability of G-CSF. SDS-PAGE with subsequent silver staining of: G-CSF from VPGs treated with triton (lane 1), G-CSF extracted from VPGs by dichloromethane (lane 2), G-CSF extracted from VPGs by chloroform (lane 3); G-CSF extracted from VPGs by ethanol (lane 4), G-CSF standard (lane 5), and molecular standard marker (lane 6).
**Figure 5****:**
   Cumulative release of EPO from VPGs with various lipid contents (egg PC Lipoid E80; 30% to 55%) (mean± standard deviation, n = 3).
**Figure 6****:**
   Cumulative release of EPO from VPGs with various lipid compositions: 50% egg PC (LIPOID E80) alone or a combination of egg PC (LIPOID E80) with DPPA in a ratio of 9:1 or of egg PC (LIPOID E80) and DOTAP in a ratio of 8:2 (mean± standard deviation, n = 3).
**Figure 7****:**
   Cumulative release of GCSF from various VPG formulations: Lipoid E80 in a concentration of 40%, 45% and 49% as well as Phospholipon 85G in a concentration of 49% (mean± standard deviation, n = 3).
**Figure 8****:**
   Stability of EPO within the release cells at 37°C. The protein was either incorporated in a VPG formulation (500mg/g) or dissolved in PBS buffer (pH 7.4). The protein concentration was 4.0mg/g, respectively.

### DETAILED DESCRIPTION OF THE INVENTION

The task in accordance with the invention is to find a way to administer proteinaceous substances that have known biological effects and to avoid the known disadvantages of alternative sustained release applications (for example, the disadvantage of accepting only relatively low drug loads, having an undesired burst release profile, and stability problems of the drug during manufacturing, release and storage).

Therefore, it is a goal of this invention to make available a pharmaceutical composition comprising at least one proteinaceous substance that enables an overall low, but at the same time sufficiently high, local dosage of pharmaceutically active compounds in a predetermined region of the body. Another goal of the invention is to make available a composition that can be easily administered anywhere on or inside the body, especially by direct injection of the composition into or onto a predetermined region of the body. Another goal of the invention is to make available a composition that can be prepared as a sterile product and thus can be applied as a therapeutic agent by injection. Finally, a goal of the invention is also to make available a composition that produces a proteinaceous substance depot in the body of a subject, from which the substance is continuously released, so that better bioavailability and longer half-lives are also achieved by comparison with systemic administration.

In accordance with the invention, these goals are achieved by making available a liposome system for administration of at least one proteinaceous substance. Surprisingly, it turned out that by enclosing proteinaceous substances in liposomes of a certain composition and formulating these liposomes in suitable galenical systems (such as a "vesicular phospholipid gel") by dual asymmetric centrifugation, said goals can be attained. The scalable method for encapsulation of the at least one proteinaceous substance disclosed herein for the preparation and loading of vesicular phospholipid gels proved to be especially advantageous because of its high efficiency, while at the same time having extremely mild process conditions.

The present invention is based on the unexpected finding that the pharmaceutical composition of the present invention comprising a vesicular phospholipid gel, the gel comprising tightly packed liposomes having a vesicular structure and at least one proteinaceous substance as the pharmaceutically active compound in encapsulated form, is ideally suited for sustained release of biologically active proteinaceous substances (i.e. proteins, polypeptides or peptides). The inventive composition is thus particularly suited for immunotherapy, for stimulating selective tissue regeneration and for the delivery of growth hormones and other peptides and proteins. In comparison to preparations known from the art, the inventive composition not only exhibits a substantially continuous release rate of the biologically active proteinaceous substance over a couple of days up to months, but also reliably provides for a continuous release rate lacking an undesired burst release.

It has surprisingly been found that the method according to the present invention allows the cheap and effective incorporation of expensive and sensitive substances, such as biologically active proteins, peptides and polypeptides, into liposomes of a vesicular phospholipid gel. In contrast to the liposome preparation techniques described in the art, no outstanding technological know-how is required, the danger for the environment resulting from the use of hazardous substances such as certain organic solvents is limited, the equipment required for the inventive method is affordable and easy to store and the inventive method does not unnecessarily strain the expensive and sensitive substances to be incorporated into the liposomes. Furthermore, due to the fact that the manufacturing is basically a one-step process within a single container no material (neither excipients nor drug) is lost during production and high drug loads are possible.

Due to the controlled release of the proteinaceous substance from the composition of the invention, it is possible to obtain a controlled rate of release or a controlled pulsatile release of the proteinaceous substance over a specific period of time. Adherence to a strict dosage regimen, e.g. requiring administration of a drug at set intervals up to several times a day, may therefore be dispensed with. Furthermore, it is possible to include two or more different proteinaceous substances in the composition of the invention, adapted to be released at different concentrations and/or intervals, thus making it easier for patients to follow a prescribed regimen.

An additional advantage of the composition of the invention is that it may be produced by relatively simple, fast, reproducible, and inexpensive methods, i.e. by centrifugation, as will be explained in more detail below. Furthermore, the composition allows for the incorporation of high concentrations of the proteinaceous substance relative to the composition's size. This is obviously a great advantage, notably when the composition is to be used for the delivery of a pharmaceutically active substance, since it allows for the delivery of the required amount of the proteinaceous substance without the composition being unnecessarily large.

The composition of the present invention thus possesses one or more of the following desirable characteristics: (1) biocompatible (i.e., substantially non-toxic), (2) non-allergenic (i.e., produce no or tolerable levels of immune and inflammatory responses), (3) of non-animal origin, (4) stable at refrigerator or at room temperature, (5) readily syringeable and/or injectable so that they can be introduced to a desired soft tissue site using a catheter or an injection needle, (6) persistent at the site of administration, preferably adhering to the soft tissue into which they have been administered, (7) tough and plastic (i.e. capable of bearing loads without undergoing excessive or permanent deformation), (8) intrudable (i.e., form a relatively dispersed, irregularly shaped mass on or within the tissue where the composition has been introduced), (9) bio-absorbable, (10) capable of providing sustained local drug delivery, and (11) excellent stability of the drug during manufacturing and release.

In the following, some of the used terms will be defined in greater detail:
Where the term "comprising" is used in the present description and claims, it does not exclude other elements or steps. For the purposes of the present invention, the term "consisting of" is considered to be a preferred embodiment of the term "comprising of". If hereinafter a group is defined to comprise at least a certain number of embodiments, this is also to be understood to disclose a group which preferably consists only of these embodiments.
Where an indefinite or definite article is used when referring to a singular noun e.g. "a" or "an", "the", this includes a plural of that noun unless something else is specifically stated.

As used herein, "preparation", "formulation" and "composition" may be used interchangeably herein, and refer to a combination of two or more elements, or substances.

As used herein, "active agent", "bioactive agent", "pharmaceutically active agent" and "pharmaceutically active compound" may be used interchangeably to refer to an agent, substance or compound that has measurable specified or selected physiologic activity when administered to a subject in a significant or effective amount. It is to be understood that the term "drug" is expressly encompassed by the present definition as many drugs known to have specific physiologic activities. These terms of art are well-known in the pharmaceutical and medicinal arts. Examples of drugs useful in the present invention include without limitation, proteins, peptides, and polypeptides.

As used herein, "effective amount," and "sufficient amount" may be used interchangeably and refer to an amount of an ingredient which, when included in a composition, is sufficient to achieve an intended compositional or physiological effect. Thus, a "therapeutically effective amount" refers to a non-toxic, but sufficient amount of a pharmaceutically active compound, to achieve therapeutic, preservative or diagnostic results in treating a condition for which the pharmaceutically active compound is known to be effective. It is understood that various biological factors may affect the ability of a substance to perform its intended task. Therefore, an "effective amount" or a "therapeutically effective amount" may be dependent in some instances on such biological factors. Further, while the achievement of therapeutic effects may be measured by a physician or other qualified medical personnel using evaluations known in the art, it is recognized that individual variation and response to treatments may make the achievement of therapeutic effects a subjective decision. The determination of an effective amount is well within the ordinary skill in the art of pharmaceutical sciences and medicine.

A "solvent" is a liquid which can dissolve gases, other liquids or solid matter without chemical reactions between dissolved matter and dissolving liquid taking place.

As used herein, "subject" refers to a mammal that may benefit from the administration of a composition or method as recited herein. Most often, the subject will be a human but can be of other animals such as for example dogs and cats.

As used herein, "administration," and "administering" refer to the manner in which an active agent, or composition containing such, is presented to a subject.

As used herein, "depot" refers to a temporary matrix inside a biological tissue or system, which includes a pharmaceutically active compound that is released from the matrix over a period of time.

A "vesicular phospholipid gel" (VPG) within the meaning of the present invention is a liposomal formulation which is viscous, i.e., no longer free-flowing, due to a high content of phospholipid (preferably at least 30 wt-%) and thus has a high liposome (vesicle) density. A VPG has to be differentiated from liposome-containing gels where the gel properties (viscosity, prevention of free flowing) are not caused by a high liposome density but by a gel-forming component such as, e.g., polyacrylamide. However, it shall not be excluded that a VPG could be further rigidified by the addition of a gel forming agent.

Within the context of the present invention, the "enclosure efficiency" is the ratio (percentage) of the amount of the proteinaceous substance (e.g., of a protein) which is included in the liposomes, associated to the liposomes or incorporated in the gel between liposomal vesicles, or could also be present in a cavity inside the VPG that would not meet the definition of a regular liposome, to the total amount of the respective substance used for the preparation.

Concentrations, amounts, solubilities, and other numerical data may be expressed or presented herein in a range format. It is to be understood that such a range format is used merely for convenience and brevity and thus should be interpreted flexibly to include not only the numerical values explicitly recited as the limits of the range, but also to include all the individual numerical values or sub-ranges encompassed within that range as if each numerical value and sub-range is explicitly recited. As an illustration, a numerical range of "about 1 to about 5" should be interpreted to include not only the explicitly recited values of about 1 to about 5, but also include individual values and sub-ranges within the indicated range. Thus, included in this numerical range are individual values such as 2, 3, and 4 and sub-ranges such as from 1-3, from 2-4, and from 3-5, etc. This same principle applies to ranges reciting only one numerical value. Furthermore, such an interpretation should apply regardless of the breadth of the range or the characteristics being described.

Within the context of the present invention, "mean size" or "mean particle size" designates the median of the liposome size, i.e., the liposome diameter where 50% of the liposomes are smaller and 50% of the liposomes are larger than the stated value. Usually, this corresponds to the maximum of a Gaussian size distribution.

In the following, the present invention will be described with respect to particular embodiments and with reference to certain drawings but the invention is not limited thereto but only by the claims.

In a first aspect, the present invention relates to a pharmaceutical composition for sustained release of a pharmaceutically active compound, comprising a vesicular phospholipid gel having a viscosity of at least 3.0 Pa·s at 25°C at a shear rate of 38 s⁻¹ comprising tightly packed liposomes having a vesicular structure, wherein
(i) the vesicular phospholipid gel comprises at least one proteinaceous substance as the pharmaceutically active compound in encapsulated form, the at least one proteinaceous substance being a biologically active protein, peptide or polypeptide, wherein the at least one proteinaceous substance has a molecular weight of at least 3,400 g/mol; and
(ii) the gel exhibits a substantially continuous release rate of the at least one proteinaceous substance over a period of at least 200 hours when exposed at about 37°C to an aqueous medium.

Accordingly, the pharmaceutical composition of the present invention comprises a vesicular phospholipid gel that comprises tightly packed liposomes having a vesicular structure, i.e. the gel-structure is made up by tightly packed phospholipid vesicles (liposomes). The term "vesicular phospholipid gels" as used herein is used for semisolid (gel-like or cream-like), aqueous phospholipid-dispersions, where the lipid phase consists of vesicles or liposomes. In contrast to other hydrogels, in VPGs, tightly packed vesicles rather than macromolecular thickeners cause the gel-like rhelogical behavior. In other words, in VPGs, the vesicles are so tightly packed that the aqueous space between the vesicles is reduced to a minimum, which gives rise to steric interactions between neighbor-vesicles and thus semisolid consistency. It is to be understood that VPGs are not be confused with so called "liposome gels", where a hydrophilic thickener such as polyacrylate is forming the gel-matrix and some liposomes are embedded in this hydrogel-matrix.

It has been surprisingly found that the inventive composition is particularly suited for sustained release of a pharmaceutically active proteinaceous compound. Time release technology also known as sustained-release (SR), extended-release (ER, XR, or XL), time-release or timed-release, controlled-release (CR), or continuous-release (CR or Contin) compositions are formulated to dissolve slowly and release a pharmaceutically active compound over time. The advantages of sustained-release compositions are that they can be applied less frequently than instant-release formulations of the same pharmaceutically active compound, and that they keep steadier levels of the pharmaceutically active compound in the bloodstream when administered to a patient.

According to the present invention, the above-referenced at least one pharmaceutically active proteinaceous compound is present in the pharmaceutical composition in any amount being effective to achieve the desired pharmacological effect, such as for example to stimulate selective tissue regeneration in the treatment of surgical defects in the course of surgical interventions when administered to a patient. Effective amounts are generally chosen in accordance with a number of factors, e.g., the age, size and general condition of the patient and the medical condition being treated, and determined by a variety of means, for example, dose ranging trials, well known to, and readily practiced by persons of ordinary skill in art given the teachings of this invention.

Typically, the amount of each active pharmaceutically active compound in the inventive pharmaceutical composition is in the range of 0.001g to 15 g in 100 g of the composition (i.e. 0.001% to 15% by weight based on the total weight (w/w) of the composition). This amount of active ingredient corresponds to a concentration of 0.001 mg to 150 mg per g of the composition.

According to the present invention, the vesicular phospholipid gel comprises the at least one proteinaceous substance as the pharmaceutically active compound in encapsulated form. Liposomes, also known as lipid vesicles, are completely closed lipid bilayer membranes which contain an entrapped volume comprising an aqueous medium. It is known in the art that biologically active materials encapsulated within liposomes are protected to varying extend from immediate dilution or degradation. The unique ability of liposomes to entrap drugs both in the aqueous and the lipid phase make such delivery systems attractive both for hydrophilic and hydrophobic drugs. Furthermore, such encapsulation has been shown to reduce drug toxicity while retaining or even improving the therapeutic efficacy. Without wishing to be bound by any therory, the at least one proteinaceous substance according to the invention may be entrapped in the aqueous and/or the lipid phases of the liposomes, depending on the hydrophilicity of the respective proteinaceous substance, may be associated to the liposomes or incorporated in the gel between liposomal vesicles, or could also be present in a cavity inside the VPG that would not meet the definition of a regular liposome

According to the present invention, the term "proteinaceous substance" denotes a macromolecular biological compound which comprises a protein, polypeptide, peptide, glycoprotein, lipoprotein and higher ordered structures like virus envelopes (e.g. viruses or virus like particles) or a derivative thereof. The term "derivative" of a protein, polypeptide or peptide as used herein means a chemically modified biological compound wherein the chemical modification takes place at one or more functional groups of the biological compound. The derivative however is expected to retain or even to improve the biological and/or pharmacological activity of the compound from which it is derived. However, the term "proteinaceous substance" as used herein does not denote a low molecular weight biological compound. A "low molecular weight biological compound", as used herein, is a molecule comprising at least two carbon atoms, and having a molecular weight of of at least 3,400 g/mol, preferably at least 4,000 g/mol, more preferably at least 4,500 g/mol, and most preferably at least 5,000 g/mol.

With respect to the objects of the present invention, a "protein" capable of being contained in the pharmaceutical composition of the present invention includes a biologically active protein or derivatives and mutants thereof, and may be naturally occurring, recombinantly manipulated or synthesized. Also, the protein may possess a variety of modifications, such as an addition, substitution, or deletion of an amino acid or domain, or glycosylation, and is not specifically limited. Hence, as used herein, the term "proteins" also comprises lipoproteins and glycoproteins. The term "proteins" shall also include e.g. protein aggregates.

Examples of "proteins" include human growth hormone, growth hormone releasing hormone, growth hormone releasing peptide, interferons, colony stimulating factors, interleukins, macrophage activating factor, macrophage peptide, B cell factor, T cell factor, protein A, allergy inhibitor, cell necrosis glycoproteins, immunotoxin, lymphotoxin, tumor necrosis factor, tumor suppressors, metastasis growth factor, alpha-1 antitrypsin, albumin and fragment polypeptides thereof, apolipoprotein-E, erythropoietin, factor VII, factor VIII, factor IX, plasminogen activating factor, urokinase, streptokinase, protein C, C-reactive protein, renin inhibitor, collagenase inhibitor, superoxide dismutase, platelet-derived growth factor, epidermal growth factor, osteogenic growth factor, bone stimulating protein, calcitonin, insulin, atriopeptin, cartilage inducing factor, connective tissue activating factor, follicle stimulating hormone, luteinizing hormone, luteinizing hormone releasing hormone, nerve growth factors, parathyroid hormone, relaxin, secretin, somatomedin, insulin-like growth factor, adrenocortical hormone, glucagon, cholecystokinin, pancreatic polypeptide, gastrin releasing peptide, corticotropin releasing factor, thyroid stimulating hormone, monoclonal or polyclonal antibodies against various viruses, bacteria, toxins, etc., and virus-derived vaccine antigens.

The terms "polypeptide", "peptide", "oligopeptide" and "protein" are used interchangeably herein to refer to a polymer or oligomer of consecutive amino acid residues. As used herein, the term "amino acid" refers to a list of abbreviations, letters, characters or words representing amino acid residues. Amino acids may be referred to herein by either their commonly known three letter symbols or by the one-letter symbols recommended by the IUPAC-IUB Biochemical Nomenclature Commission.

One convention is that those peptide chains that are short enough to be made synthetically from the constituent amino acids are called "peptides" rather than "proteins". However, with the advent of better synthetic techniques, peptides as long as hundreds of amino acids can be made, including full proteins like ubiquitin. Native chemical ligation has given access to even longer proteins, so this convention seems to be outdated. Another convention places an informal dividing line at approximately 50 amino acids in length (some people claim shorter lengths). According to this convention, the term "polypeptide" denotes a single linear chain of amino acids, the term "protein" denotes one or more polypeptides more than about 50 amino acids long and the term "oligopeptide" or (simply) "peptide" denotes a polypeptide less than 30-50 amino acids long.

Accordingly, the terms "protein", "peptide" and "polypeptide" are used interchangeably herein to refer to a polymer or oligomer of consecutive amino acid residues, the term "neuropeptide" is used herein to denote a peptide that is active in association with neural tissue and the term "peptide hormone" is used herein to denote a peptide that acts as a hormone.

Within the context of the present invention, proteins capable of being contained in the pharmaceutical composition of the present invention include antibodies (also known as immunoglobulins, abbreviated Ig), which are gamma globulin proteins that are found in blood or other bodily fluids of vertebrates, and are used by the immune system to identify and neutralize foreign objects, such as bacteria and viruses. They are typically made of basic structural units-each with two large heavy chains and two small light chains-to form, for example, monomers with one unit, dimers with two units or pentamers with five units. Antibodies are produced by a kind of white blood cell called a B cell. There are several different types of antibody heavy chains, and several different kinds of antibodies, which are grouped into different isotypes based on which heavy chain they possess. Although the general structure of all antibodies is very similar, a small region at the tip of the protein is extremely variable, allowing millions of antibodies with slightly different tip structures to exist. This region is known as the hypervariable region. Each of these variants can bind to a different target, known as an antigen. This huge diversity of antibodies allows the immune system to recognize an equally wide diversity of antigens. The unique part of the antigen recognized by an antibody is called an epitope. These epitopes bind with their antibody in a highly specific interaction, called induced fit, that allows antibodies to identify and bind only their unique antigen in the midst of the millions of different molecules that make up an organism. Recognition of an antigen by an antibody tags it for attack by other parts of the immune system. Antibodies can also neutralize targets directly by, for example, binding to a part of a pathogen that it needs to cause an infection.

"Targeted" monoclonal antibody therapy is often employed to treat diseases such as rheumatoid arthritis, multiple sclerosis, psoriasis, and many forms of cancer including non-Hodgkin's lymphoma, colorectal cancer, head and neck cancer and breast cancer. Some immune deficiencies, such as X-linked agammaglobulinemia and hypogammaglobulinemia, result in partial or complete lack of antibodies. These diseases are often treated by inducing a short term form of immunity called passive immunity. In preferred embodiments of the invention, the proteinaceous substance is a (monoclonal) antibody selected from the group consisting of abciximab, adalimumab, alemtuzumab, basiliximab, bevacizumab, cetuximab, daclizumab, eculizumab, efalizumab, ibritumomab tiuxetan, infliximab, muromonab-CD3, natalizumab, omalizumab, palivizumab, panitumumab, ranibizumab, gemtuzumab ozogamicin, rituximab, tositumomab and trastuzumab.

Within the context of the present invention, proteins capable of being contained in the pharmaceutical composition of the present invention include growth factors. As used herein, the term "growth factor" refers to a naturally occurring protein capable of stimulating cellular growth, proliferation and cellular differentiation. Growth factors are important for regulating a variety of cellular processes and typically act as signaling molecules between cells. Examples of growth factors are cytokines and hormones that bind to specific receptors on the surface of their target cells. They often promote cell differentiation and maturation, which varies between growth factors. For example, bone morphogenic proteins stimulate bone cell differentiation, while fibroblast growth factors and vascular endothelial growth factors stimulate blood vessel differentiation (angiogenesis). In preferred embodiments of the invention, the proteinaceous substance is a growth factor selected from the group consisting of epidermal growth factors, neuregulins, fibroblast growth factors, cytokines, hematopoietic cytokines, insulin-like growth factors, interleukins, neurotrophic factors, platelet derived growth factors, transforming growth factors, tumor necrosis factors and VEGF.

EGF is the founding member of the EGF-family of proteins. Members of this protein family have highly similar structural and functional characteristics. In the context of the present invention, epidermal growth factors include epidermal growth factor (EGF), Heparin-binding EGF-like growth factor (HB-EGF), transforming growth factor-α (TGF-α), Amphiregulin (AR), Epiregulin (EPR), Epigen, Betacellulin (BTC), neuregulin-1 (NRG1), neuregulin-2 (NRG2), neuregulin-3 (NRG3), and neuregulin-4 (NRG4).

The Neuregulins are a family of four structurally-related proteins that are part of the EGF family of proteins. These proteins have been shown to have diverse functions in the development of the nervous system. The neuregulin family includes: Neuregulin-1 (NRG1), with numerous discovered isoforms stemming from alternative splicing: Type I NRG1 (alternative names: Heregulin, NEU differentiation factor (NDF), or acetylcholine receptor inducing activity (ARIA)); Type II NRG1 (alternative name: Glial Growth Factor-2 (GGF2)); Type III NRG1 (alternative name: Sensory and motor neuron-derived factor (SMDF)); Type IV NRG1; Type V NRG1; Type VI NRG1; as well as Neuregulin-2 (NRG2); Neuregulin-3 (NRG3); and Neuregulin-4 (NRG4).

Fibroblast growth factors, or FGFs, are a family of growth factors involved in angiogenesis, wound healing, and embryonic development. The FGFs are heparin-binding proteins and interactions with cell-surface associated heparan sulfate proteoglycans have been shown to be essential for FGF signal transduction. FGFs are key-players in the processes of proliferation and differentiation of wide vareity of cells and tissues. According to the present invention, the FGF family includes members FGF1 through FGF10 (all bind fibroblast growth factor receptors (FGFRs); FGF1 is also known as "Acidic", and FGF2 is also known as basic fibroblast growth factor); members FGF11, FGF12, FGF13, and FGF14 (also known as FGF homologous factors 1-4 (FHF1-FHF4), have been shown to have distinct functional differences compared to the FGFs; although these factors possess remarkably similar sequence homology, they do not bind FGFRs and are involved in intracellular processes unrelated to the FGFs; this group is also known as "iFGF"); members FGF16 through FGF23 (in contrast to the local activity of the other FGFs, FGF15/FGF19, FGF21 and FGF23 have more systemic effects).

Cytokines are a category of signaling molecules that, like hormones and neurotransmitters, are used extensively in cellular communication. The term cytokine encompasses a large and diverse family of polypeptide regulators that are produced widely throughout the body by cells of diverse embryological origin. Cytokine is a general name; other names include lymphokine (cytokines made by lymphocytes), monokine (cytokines made by monocytes), chemokine (cytokines with chemotactic activities), and interleukin (cytokines made by one leukocyte and acting on other leukocytes). Cytokines may act on the cells that secrete them (autocrine action), on nearby cells (paracrine action), or in some instances on distant cells (endocrine action). The largest group of cytokines stimulates immune cell proliferation and differentiation. This group includes Interleukin 1 (IL-1), which activates T cells; IL-2, which stimulates proliferation of antigen-activated T and B cells; IL-4, IL-5, and IL-6, which stimulate proliferation and differentiation of B cells; Interferon gamma (IFNγ), which activates macrophages; and IL-3, IL-7 and Granulocyte Monocyte Colony-Stimulating Factor (GM-CSF), which stimulate hematopoiesis. Other groups of cytokines include interferons and chemokines. Interferons IFNα and IFNβ inhibit virus replication in infected cells, while IFNγ also stimulates antigen-presenting cell MHC expression. Chemokines attract leukocytes to infection sites. Chemokines have conserved cysteine residues that allow them to be assigned to four groups. The groups, with representative chemokines, are C-C chemokines (RANTES, MCP-1, MIP-1a, and MIP-1b), C-X-C chemokines (IL-8), C chemokines (Lymphotactin), and CXXXC chemokines (Fractalkine). Some cytokines are predominantly inhibitory. For example, IL-10 and IL-13 inhibit inflammatory cytokine production by macrophages. The cytokines further include the family of adipokines or adipocytokines secreted by adipose tissue, the family including chemerin, interleukin-6 (IL-6), plasminogen activator inhibitor-1 (PAI-1), retinol binding protein 4 (RBP4), tumor necrosis factor-alpha (TNFα) and visfatin. Within the context of the present invention, the cytokine family thus includes GM-CSF, IL-1α, IL-1β, IL-2, IL-3, IL-4, IL-5, IL-6, IL-7, IL-8, IL-10, IL-12, IFN-α, IFN-β, IFN-γ, MIP-1α, MIP-1β, TGF-β, TNFα, TNF-β.

Hematopoietic cytokines (also called hematopoietic growth factors) are produced by many different cell types including those not belonging to the hematopoietic system. These factors are either secreted or they exist in membrane-bound or matrix-associated forms. They may have different modes of action also, such as autocrine, paracrine, or juxtacrine growth control. Production of hematopoietic factors is regulated strictly, i.e., they are synthesized by activated cells under certain conditions rather than being produced constitutively all the time. "Hematopoietic growth factors", as used herein, include the various colony stimulating factors (see: CSF, including G-CSF, GM-CSF, M-CSF), Epo, SCF (stem cell factor), SCPF (stem cell proliferation factor), various Interleukins (IL1, IL3, IL4, IL5, IL6, IL11, IL12), LIF, TGF-beta, MIP-1-alpha, TNF-alpha, also many other low molecular weight factors (see also: AcSDKP; pEEDCK, thymic hormones, regulatory peptide factors), and several other proteins identified initially by some biological activities that have nothing to do with hematopoiesis.

The insulin-like growth factors (IGFs) are polypeptides with high sequence similarity to insulin. IGFs are part of a complex system that cells use to communicate with their physiologic environment. This complex system (often referred to as the IGF "axis") consists of two cell-surface receptors (IGF1R and IGF2R), two ligands (IGF-1 and IGF-2), a family of six high-affinity IGF binding proteins (IGFBP 1-6), as well as associated IGFBP degrading enzymes, referred to collectively as proteases. Within the context of the present invention, the insulin-like growth factor family includes IGF-1 and IGF-2.

Interleukins are a group of cytokines (secreted signaling molecules) that were first seen to be expressed by white blood cells as a means of communication. It has been found that interleukins are produced by a wide variety of body cells. The function of the immune system depends in a large part on interleukins, and rare deficiencies of a number of them have been described, all featuring autoimmune diseases or immune deficiency. Within the context of the present invention, the interleukin family includes IL-1, IL-2, IL-3, IL-4, IL-5, IL-6, IL-7, IL-8, IL-9, IL-10, IL-11, IL-12, IL-13, IL-14, IL-15, IL-16, IL-17, IL-18, IL-19, IL-20, IL-21, IL-22, IL-23, IL-24, IL-25, IL-26, IL-27, IL-28, IL-29, IL-30, IL-31, IL-32, IL-33, and IL-35.

Neurotrophins are a family of proteins that induce the survival, development and function of neurons. They belong to a class of growth factors, secreted proteins, which are capable of signaling particular cells to survive, differentiate, or grow. Growth factors such as neurotrophins that promote the survival of neurons are known as neurotrophic factors. Neurotrophic factors are secreted by target tissue and act by preventing the associated neuron from initiating programmed cell death - thus allowing the neurons to survive. Neurotrophins also induce differentiation of progenitor cells, to form neurons. Within the context of the present invention, the neurotrophic factor family includes nerve growth factor (NGF), brain-derived neurotrophic factor (BDNF), neurotrophin-3 (NT-3), neurotrophin-4 (NT-4), and neurotrophin-1 (NNT1).

The platelet-derived growth factor (PDGF) is one of the numerous growth factors, or proteins that regulate cell growth and division. In particular, it plays a significant role in blood vessel formation (angiogenesis), the growth of blood vessels from already existing blood vessel tissue. Uncontrolled angiogenesis is a characteristic of cancer. There are five different isoforms of PDGF that activate cellular response through two different receptors, including ligands A (PDGFA), B (PDGFB), C (PDGFC) and D (PDGFD) and an AB heterodimer and receptors alpha (PDGFRA) and beta (PDGFRB).

The term "transforming growth factors" (sometimes referred to as tumor growth factor, or TGF) is used herein to describe two classes of polypeptide growth factors, TGFα and TGFβ. TGFα is upregulated in some human cancers. It is produced in macrophages, brain cells, and keratinocytes, and induces epithelial development. TGFβ exists in three known subtypes in humans, TGFβ1, TGFβ2, and TGFβ3. These are upregulated in Marfan's syndrome and some human cancers, and play crucial roles in tissue regeneration, cell differentiation, embryonic development, and regulation of the immune system.

As used herein, tumor necrosis factors (or the TNF-family) refers to a group of cytokines family that can cause apoptosis, including tumor necrosis factor-alpha (TNF-α) which is the most well-known member of this class, and sometimes referred to when the term "tumor necrosis factor" is used, and tumor necrosis factor-beta (TNF-β) which is a cytokine that is induced by interleukin 10.

Vascular endothelial growth factor (VEGF) is a sub-family of growth factors, more specifically of platelet-derived growth factor family of cystine-knot growth factors. They are important signaling proteins involved in both vasculogenesis (the de novo formation of the embryonic circulatory system) and angiogenesis (the growth of blood vessels from pre-existing vasculature). According to the present invention, the VEGF family includes VEGF-A, placenta growth factor (P1GF), VEGF-B, VEGF-C and VEGF-D.

Within the context of the present invention, proteins capable of being contained in the pharmaceutical composition of the present invention further include peptide hormones. Peptide hormones are a class of peptides that are secreted into the blood stream and have endocrine functions in living animals. Like other proteins, peptide hormones are synthesized from amino acids according to an mRNA template, which is itself synthesized from a DNA template inside the cell's nucleus. Peptide hormone precursors (pre-prohormones) are then processed in several stages, typically in the endoplasmic reticulum, including removal of the N-terminal signal sequence and sometimes glycosylation, resulting in prohormones. The prohormones are then packaged into membrane-bound secretory vesicles, which can be secreted from the cell by exocytosis in response to specific stimuli. These prohormones often contain superfluous amino acid residues that were needed to direct folding of the hormone molecule into its active configuration but have no function once the hormone folds. Specific endopeptidases in the cell cleave the prohormone just before it is released into the blood stream, generating the mature hormone form of the molecule. Mature peptide hormones then diffuse through the blood to all of the cells of the body, where they interact with specific receptors on the surface of their target cells. In some embodiments of the present invention, the proteinaceous substance is a proteohormone selected from the group consisting of cytokines such as granulocyte-colony stimulating factor, interferons and interleukins; hematopoetic hormones such as erythropoietin; and peptide hormones such as follicle stimulating hormone, luteinizing hormone, gonadotropin-releasing hormone, glucagon and insulin.

The release rate of a pharmaceutically active compound from VPG can be determined by a variety of means, for example, flow-through cells, well known to, and readily practiced by persons of ordinary skill in art given the teachings of this invention. According to the present invention, the release rate may be determined *in vitro* or *in vivo*. The *in vitro* release behaviour may be measured as exemplified below.

During the last decades many different systems for modifying the release of an active drug substance from a pharmaceutical composition have been developed. Most of them aim at obtaining a zero or a first order release rate of the active substance from the composition. Zero order release rate (i.e. constant release of the active substance with time) seemed to be very difficult to obtain from a pharmaceutical composition. Without wishing to be bound by any theory, it is believed that the release of drugs from vesicular phospholipid gels in principle may happen by two mechanism: First, diffusion of the drug out of the phospholipid matrix and second, erosion of the matrix, thus releasing liposomes with encapsulated drug as well as drug that has been trapped in-between the vesicles, or any combination of both mechanisms. Which of the two mechanisms becomes rate limiting mainly depends on the composition of the vesicular phospholipid gel as well as the physicochemical properties of the proteinaceous substance. Without wishing to be bound by any theory, it is believed that that the release rate of matrix-controlled diffusion typically follows square root of time kinetics, whereas the release rate of matrix erosion typically follows slower zero-order kinetics.

The pharmaceutical composition of the present invention exhibits a substantially continuous release rate of the at least one proteinaceous substance entrapped in the liposomes over a certain period of time. The term "continuous release rate" in the context of the invention is used for a release of the proteinaceous substance that is continuous and preferably stable in quantity and rate. Accordingly, the term "continuous" means a release that occurs without interruption, and preferably without increase or abatement. The phrase "stable in quantity and rate" in this context means predictable and regular in amount and rate of emission. It is particularly preferred that the pharmaceutical composition of the present invention exhibits a substantially constant release rate of the at least one proteinaceous substance entrapped in the liposomes over a certain period of time, most preferably following zero-order kinetics. The term "constant release rate" is thus used for a release of the proteinaceous substance that is continuous and stable in quantity and rate over a certain period of time. Accordingly, the invention most preferably relates to a pharmaceutical composition which provides zero order release based on controlling the balance between matrix erosion rate and diffusion rate in the phospholipid matrix.

Accordingly, the gel comprised by the pharmaceutical composition exhibits a substantially continuous release rate of the at least one proteinaceous substance entrapped in the liposomes over a period of at least 200 hours, even more preferably at least 250 hours, and most preferably 300 hours, when exposed at about 37°C to an aqueous medium. It is particularly preferred that a substantially zero order release rate is obtained over a time period of at least 200 hours, even more preferably at least 250 hours, and most preferably 300 hours. Adherence to a strict dosage regimen, e.g. requiring administration of a drug at set intervals up to several times a day, may therefore be dispensed with. Furthermore, it is possible to include two or more different proteinaceous substances in the composition of the invention, adapted to be released at different concentrations and/or intervals, thus making it easier for patients to follow a prescribed regimen.

In other specific embodiments, the pharmaceutical composition exhibits a release of the at least one proteinaceous substance of less than 20%, preferably less than 17%, more preferably less than 15%, and most preferably less than 12% during 24 hrs when exposed at about 37°C to an aqueous medium. It is to be understood, that the term "24 hrs" refers to any continuous time section of the exposure period of the pharmaceutical composition to an aqueous medium, preferably to the first 24 hrs. The design of the pharmaceutical composition is thus based on the unexpected finding that it is possible to control the release rate from such a composition by ensuring that it does not have an undesired increased initial release or an undesired burst release.

With respect to the objects of the present invention, the term "aqueous medium" is used for an aqueous solvent (e.g. water or buffer solution). Preferably, the "aqueous medium" mainly comprises water, which may contain dissolved inorganic or organic salts, acids or bases, for example, for setting and achieving a specific pH value or a specific osmotic pressure. However, the aqueous medium may also comprise aqueous solutions of saccharides, amino acids or surfacants or mixtures of water with other solvents like alcohols, diols or triols. A buffered solution is an aqueous solution comprising a mixture of a weak acid and its conjugate base or a weak base and its conjugate acid. It has the property that the pH of the solution changes very little when a small amount of acid or base is added to it. Buffered solutions are used as a means of keeping pH at a nearly constant value in a wide variety of chemical applications. Suitable buffer systems e.g. include Phosphate, Citrate, Acetate, Succinate, Maleate, Histidine, TAPS, Bicine, Tris, Tricine, HEPES, TES and MOPS. These and alternative buffer systems are well known to persons of ordinary skill in art given the teachings of this invention. Suitable is for example a 40 mM phosphate-buffer at pH 7.4.

The term "phospholipids" refers to lipid molecules containing one or more phosphate groups, including those derived from either glycerol (phosphoglycerides, glycerophospholipids) or sphingosine (sphingolipids). They include polar lipids, and certain phospholipids that are of great importance for the structure and function of cell membranes and are the most abundant of membrane lipids. In certain embodiments, phospholipids are triglyceride derivatives in which one fatty acid has been replaced by a phospharylated group and one of several nitrogen-containing molecules. The fatty acid chains are hydrophobic (as in all fats). However, the charges on the phosphorylated and amino groups make that portion of the molecule hydrophilic. The result is an amphiphilic molecule.

Hence, with respect to the objects of the present invention, a phospholipid is an amphipathic substance which generally contains a hydrophobic group comprising a long chain alkyl group and a hydrophilic group comprising a phosphoric acid group in one molecule. Exemplary phospholipids which may be used in the present invention include glycerophospholipids such as phosphatidylcholine (=lecithin), phosphatidylglycerol, phosphatidic acid, phosphatidylethanolamine, phosphatidylserine, and phosphatidylinocitol; sphingophospholipids such as sphingomyelin (Sphingomyelin); natural or synthetic diphosphatidyl phospholipids such as cardiolipin, and derivatives thereof; and any of such phospholipid which has been hydrogenated by a method commonly used in the art (for example, hydrogenated soybean phosphatidyl choline). The phospholipids as mentioned above is hereinafter occasionally referred to as "phospholipids". The phospholipid component may contain one or more phospholipids, and is in the range from about 10% to about 90% of the total weight of the composition. In preferred embodiments of the invention, the phospholipid is selected from the group consisting of phosphatidylcho lines, phosphatidylethanolamines, phosphatidylinosites, phosphatidylserines, cephalines, phosphatidylglycerols and mixtures of several such phospholipids. In specific embodiments of the invention, a blend of phosphatidylcho lines and/or phosphatidylglycerols can be used as the phospholipid.

Phospholipids are available from naturally occurring sources or by organic synthesis. Lecithin is a naturally occurring mixture of the diglycerides of stearic, palmitic, and oleic acids, linked to the choline ester of phosphoric acid, commonly called phosphatidylcholine, which is the most abundant and structurally most important phospholipid. According to the United State Pharmacopoeia (USP), lecithin is a non-proprietary name describing a complex mixture of acetone-insoluble phospholipids, which consists chiefly of phosphotidylcholine, phosphotidylethanolamine, phosphotidylserine and phosphotidylinositol, combined with various amounts of other substances such as triglycerides, fatty acids, and carbohydrates. The composition of lecithin and hence its physical properties vary depending upon the source of the lecithin and phospholipid composition, e.g., phosphotidylcholine content, etc. The commercially available lecithin products have two primary sources: egg yolk and soybean.

One source of phospholipid materials suitable for incorporation into the compositions of the invention is soy lecithin of high purity, i.e., free from allergenic, inflammatory agents or agents that cause other deleterious biological reactions, which is qualified for use in injectable products. Such injectable forms of soy lecithin are commercially available in the brand names of Phospholipon® by Phospholipid GmbH, Lipoid® by Lipoid GmbH, Epikuron® by Degussa. These refined soy lecithin products may contain different concentrations of phosphotidylcholine (PC content) ranging from 30% to 100%. By combining lecithin products of different PC contents, it is possible to vary the consistency of the VPG and persistence in the tissue.

Another source of phospholipids is the hydrogenated lecithin of soy or egg origins. Hydrogenation saturates the double bonds on the fatty acid side chains of the lecithin molecules. The resulted saturated fatty acids are less sensitive to the oxidation or enzymatic degradation. A composition comprising a hydrogenated lecithin is thus more stable chemically and degrades slower in the tissue than its naturally form. Examples of commercially available hydrogenated lecithin of injectable grade include Phospholipon® 90H, 100H by Phospholipid GmbH and LIPOID E PC-3 and LIPOID E PS-3, LIPOID S PC-3, LIPOID S PG-3, LIPOID S PA-3, and LIPOID S PE-3 from Lipoid GmbH.

Suitable phospholipids for use in the present compositions include phospholipid blends such as those sold under the trade names Lipoid S 20 S, Lipoid S 75, Lipoid S 100, Lipoid S 100-3, Lipoid S 75-3N, Lipoid SL 80, Lipoid E80, and Lipoid SL 80-3, which are commercially available from Lipoid; Phospholipon 85 G, Phospholipon 80, Phospholipon 80 H, Phospholipon 90 G, Phospholipon 90 H, Phospholipon 90 NG, Phospholipon 100 H, Phosal 35B, Phosal 50G, Phosal 50SA, Phosal 53MCT, and Phosal 75SA, which are commercially available from Phospholipon, Cologne Germany; Alcolec Z-3 available from American Lecthin Company, Oxford CT; Emulfluid F30, Emulfluid, Lipotin NE, Lipotin 100, Lipotin SB, Lipotin 100J, Lipotin H, Lipotin NA, Lipotin AH, and Lipopur, which are commercially available from Cargill (Degussa Texturant Systems); Terradrill V 408 and Terradrill V 1075, which are commercially available from Cognis; Yellowthin 100, Yellowthin 200, Lecistar Sun 100, and Yellowthin Sun 200, which are commercially available from Sternchemie; and Lanchem PE-130K available from Lambent Technologies, Gurnee, IL.

The VPG of the invention may comprise one or more additional lipids including neutral, cationic and anionic lipids. Hence, in further specific embodiments, the pharmaceutical composition further comprises a lipid selected from the group consisting of fatty acids, monoglycerides, diglycerides, triglycerides, sorbitan fatty acid esters, sphingolipids, cholesterol, waxes, and salts and derivatives thereof.

Cationic lipids for use in the present compositions include N,N-dioleyl-N,N-dimethylammonium chloride ("DODAC"); N-(2,3-dioleyloxy)propyl-N,N--N-triethylammonium chloride ("DOTMA"); N,N-distearyl-N,N-dimethylammonium bromide ("DDAB"); N-(2,3-dioleoyloxy)propyl)-N,N,N-trimethylammonium chloride ("DOTAP"); 3β-(N-(N',N'-dimethylaminoethane)-carbamoyl)cholesterol ("DC-Chol"), N-(1-(2,3-dioleyloxy)propyl)-N-2-(sperminecarboxamido)ethyl)-N,N-dimethyl- ammonium trifluoracetate ("DOSPA"), dioctadecylamidoglycyl carboxyspermine ("DOGS"), 1,2-dileoyl-sn-3-phosphoethanolamine ("DOPE"); and N-(1,2-dimyristyloxyprop-3-yl)-N,N-dimethyl-N-hydroxyethyl ammonium bromide ("DMRIE"). Additionally, a number of commercial preparations of cationic lipids can be used, such as LIPOFECTIN (including DOTMA and DOPE, available from GIBCOBRL), LIPOFECTAMINE (comprising DOSPA and DOPE, available from GIBCO/BRL), and TRANSFECTAM (comprising DOGS, in ethanol, from Promega Corp.).

Anionic lipids for use in the present compositions include phosphatidylglycerol, cardiolipin, diacylphosphatidylserine, diacylphosphatidic acid, N-dodecanoyl phosphatidylethanoloamine, N-succinyl phosphatidylethanolamine, N-glutaryl phosphatidylethanolamine, lysylphosphatidylglycerol, dipalmitoylphosphatidic acid and other anionic modifying groups joined to neutral lipids.

Any of a number of neutral lipids can also be included, referring to any of a number of lipid species which exist either in an uncharged or neutral zwitterionic form at physiological pH, including diacylphosphatidylcholine, diacylphosphatidylethanolamine, ceramide, sphingomyelin, cephalin, cholesterol, cerebrosides, and diacylglycerols.

With respect to the objects of the present invention, liposomes may be giant unilamellar vesicles (GUVs), large unilamellar vesicles (LUVs), multilamellar vesicles (MLVs) and small unilamellar vesicles (SUVs). The particle size of liposomes is varied as >=1000 nm for GUV, 100 to 1000 nm for LUV, 200 to 5000 nm for MLV, and <=100 nm for SUV. Liposomes smaller than about 20 nm are physically untenable, while liposomes larger than about 1,000 nm in diameter tend to be unstable and aggregate over time. In many therapeutic applications, and particularly in systemic delivery and tissue and cell targeting, liposome size is a critical parameter of therapeutic effectiveness. The liposome partice size can be determined by a variety of means, for example, photo correlation spectroscopy (PCS), well known to, and readily practiced by persons of ordinary skill in art given the teachings of this invention. In the invention, the average particle size refers to a simple (or arithmetic) average of the given number of observed vesicular particles of the contrast medium, for example, 20 vesicles. This value usually agrees with or is close to the central vesicle size which refers to a vesicle size having the highest frequencies in the vesicle size distribution. Liposomes having different sizes may be manufactures by changing the parameters of the dual asymmetric centrifuge (e.g., centrifugation time and speed). It has been surprisingly found that the achievement of a certain mean particle size or particle size distribution is of less importance for the sustained release of pharmaceutically active compounds from homogenous VPGs.

The phospholipid component content in the VPGs according to the present invention is from 200 to 600 mg/g VPG, preferably from 300 to 600 mg/g VPG, particularly preferred from 400 to 600 mg/g VPG. In preferred embodiments of the present invention, the pharmaceutical composition has a phospholipid content of at least 30 wt-% (300 mg/g VPG), more preferably at least 40 wt-% (400 mg/g VPG), even more preferably at least 45 wt-% (450 mg/g VPG), and most preferably at least 50 wt-% (500 mg/g VPG). In specific embodiments of the invention, the weight ratio between the phospholipid and the at least one proteinaceous substance is between about 1000:1 and 5:1, preferably between 500:1 and 25:1, more preferably between 250:1 and 40:1, and even more preferably between 150:1 and 50:1. In a particularly preferred embodiment of the invention, the weight ratio between the phospholipid and the at least one proteinaceous substance is about 100:1. The inventive composition has the advantageous effect that it that can be easily administered anywhere on or inside the body, especially by direct injection of the composition into a predetermined region of the body, thus producing a proteinaceous substance depot in the body of a subject, from which the substance is continuously released, so that better bioavailability and longer half-lives are also achieved by comparison with systemic administration.

The liposome gel composition of the invention is characterized by a high viscosity and may be in the form of fluid, viscous, soft gel, a semi-solid gel, a hard gel, or a rubbery gel. In the context of the invention, "high-viscosity" or "gel" or "gel-like" refers to a viscous, relatively non-flowable gel consistency which can be applied by squeezing from a tube or syringe, but which is sufficiently non-flowable, once applied, to be retained in bolus form at a wound or incision site for at least several hours. The rheology behavior of the inventive VPGs may be studied by a variety of means, for example, by rotational viscometer or texture analysis (as exemplified below), well known to, and readily practiced by persons of ordinary skill in art given the teachings of this invention. In preferred embodiments of the present invention, the gel has a viscosity (test system: PP 50, rotation plate; temperature: 25°C; shear rate: 10-100 1/s) of at least 0.2 Pa·s, more preferably 3.0 Pa·s, even more preferably at least 5.0 Pa·s, and most preferably at least 7.0 Pa·s, at 25°C at a shear rate of about 38 s⁻¹ (∼37.9 s⁻¹). In further preferred embodiments of the present invention, the maximum force of the inventive VPGs determined by texture analysis (test system: TA, XT plus, Stable Micro Systems, UK; microprobe of 4 mm in a gel volume of 1.5 ml; test speed 0.50 mm/s; test distance 4.000 mm; gel strength represented as the maximum force by the average of 3 parallels) is larger than 3.2 g, more preferably larger than 5 g, and most preferably larger than 10 g. It has been surprisingly found that the inventive compositions having the above rheology behavior are readily syringeable and/or injectable so that they can be introduced to a desired tissue site, body orifices or body surface using a catheter or a needle, are persistent at the site of administration, preferably adhering to the tissue into which they have been administered, form a relatively dispersed, irregularly shaped mass within the tissue where the composition has been introduced, and are therefore capable of providing sustained local drug delivery. Without wishing to be bound by any theory, it is believed that it should be possible to achieve a desired release profile for proteinaceous substances by optimizing the formulation of the compositions with respect to its viscosity.

Accordingly, in a particularly preferred aspect, the present invention relates to a pharmaceutical composition for sustained release of a pharmaceutically active compound, comprising a vesicular phospholipid gel comprising liposomes having a vesicular structure, wherein
(i) the gel has a viscosity of at least 3.0 Pa·s at 25°C at a shear rate of about 38 s⁻¹;
(ii) the gel comprises at least one proteinaceous substance as the pharmaceutically active compound, the at least one proteinaceous substance having a molecular weight of at least 3,400 g/mol; and
(iii) the gel exhibits a substantially continuous release rate of the at least one proteinaceous substance over a period of at least 200 hours when exposed at 37°C to an aqueous medium.

Preferably, the composition according to the invention also comprises at least one excipient which modifies the release of the at least one proteinaceous substance from the liposomes and/or modifies the biodegradation of the vesicular phospholipid gel and/or stabilizes the at least one proteinaceous substance during manufacturing, storage and release. Furthermore, the excipient might modify the solubility of the at least one proteinaceous substance. The excipient can e.g. be a hydrophilic, lipophilic, or amphiphilic polymer, a sugar, a polyol, a surfactant, an antioxidant, cyclodextrin, and/or a water-soluble salt or any other excipient known to achieve the above mentioned purposes.

The "hydrophilic polymer" used in the device of the present invention is a polymeric substance that is admixed with the phospholipid components. The hydrophilic polymer is present between hydrophobic lipid molecules. This hydrophilic polymer may *inter alia* protect and stabilize the proteinaceous substances, prevent denaturation of the proteinaceous substances, and induce the stable release of the proteinaceous substances. The hydrophilic polymer can prevent a protein from being denatured during the preparation of the inventive composition as well as during release after the inventive composition is administered to or into the body. Also, the hydrophilic polymer can protect a proteinaceous substance against degradation and aggregation as well as against denaturation, can enhance the *in vivo* activity of the proteinaceous substance, and/or can maintain the sustained release of the proteinaceous substance. In a preferred aspect, the present invention employs, as the hydrophilic polymer, a polyethylene glycol having a molecular weight of more than about 2,000 daltons.

A "salt" in the context of the invention is generally defined as the product formed from the neutralisation reaction of acids and bases. Salts are ionic compounds composed of cations (positively charged ions) and anions (negative ions) so that the product is electrically neutral (without a net charge). These component ions can be inorganic such as chloride, as well as organic such as acetate and monoatomic ions such as fluoride, as well as polyatomic ions such as sulfate. There are several varieties of salts. Salts that produce hydroxide ions when dissolved in water are basic salts and salts that produce hydronium ions in water acid salts. Neutral salts are those that are neither acid nor basic salts. Zwitterions contain an anionic center and a cationic center in the same molecule but are not considered to be salts. Common salt-forming cations include ammonium, calcium, iron, magnesium, potassium, pyridinium, quaternary ammonium and sodium. Common salt-forming anions (and the name of the parent acids in parentheses) include: acetate (acetic acid), carbonate (carbonic acid), chloride (hydrochloric acid), citrate (citric acid), hydroxide (water), nitrate (nitric acid), oxide (water), phosphate (phosphoric acid), succinate (succinic acid), maleate (maleinic acid), trishydroxymethylaminomethane (tris) and sulfate (sulfuric acid).

The term "sugar" as used herein means a carbohydrate. Carbohydrates include compounds such as monosaccharides, oligosaccharides, polysaccharides, glycoproteins, glycolipids and the like. Carbohydrates of the present invention also include carbohydrate-nucleoside hybrid molecules, such as carbohydrate-oligonucleotide hybrid molecules. As used herein, the term "monosaccharide" includes a compound that is the basic unit of a carbohydrate, consisting of a single sugar. Monosaccharides include glucose, glyceraldehydes, ribose, mannose, galactose and the like. As used herein, the term "oligosaccharide" refers without limitation to several (e.g., two to ten) covalently linked monosaccharide units. Oligosaccharides include disaccharides (i.e., two monosaccharide units) such as sucrose, lactose, maltose, isomaltose, cellobiose and the like. Oligosaccharides are often associated with proteins (i.e., glycoproteins) and lipids (i.e., glycolipids). Oligosaccharides form two types of attachments to proteins: N-glycosidic and O-glycosidic. As used herein, the term "polysaccharide" refers without limitation to many (e.g., eleven or more) covalently linked monosaccharide units. Polysaccharides can have molecular masses ranging well into millions of daltons. Polysaccharides include cellulose, chitin, starch, glycogen, glycosaminoglycans (e.g., hyaluronic acid, chondroitin-4-sulfate, chondroitin-6-sulfate, dermatan sulfate, keratin sulfate, heparin and the like) and the like.

As used herein, the term "polyol" is intended to include any linear, cyclic, or aromatic compound containing at least four free esterifiable hydroxyl groups. For example, suitable polyols can be selected from the following classes: saturated and unsaturated straight and branched chain linear aliphatics; saturated and unsaturated cyclic aliphatics, including heterocyclic aliphatics; or mononuclear or polynuclear aromatics, including heterocyclic aromatics. Carbohydrates and nontoxic glycols are preferred polyols. Monosaccharides suitable for use herein include, for example, mannose, galactose, arabinose, xylose, ribose, apiose, rhamnose, psicose, fructose, sorbose, tagitose, ribulose, xylulose, and erythrulose. Oligosaccharides suitable for use herein include, for example, maltose, kojibiose, nigerose, cellobiose, lactose, melibiose, gentiobiose, turanose, rutinose, trehalose, sucrose and raffinose. Polysaccharides suitable for use herein include, for example, amylose, glycogen, cellulose, chitin, insulin, agarose, zylans, mannan and galactans. Although sugar alcohols are not carbohydrates in a strict sense, the naturally occurring sugar alcohols are so closely related to the carbohydrates that they are also preferred for use herein. The sugar alcohols most widely distributed in nature and suitable for use herein are sorbitol, mannitol and galactitol. Preferred carbohydrates and sugar alcohols include trehalose, saccharose, sorbitol and mannitol.

"Surfactants", also known as tensides, are wetting agents that lower the surface tension of a liquid, allowing easier spreading, and lower the interfacial tension between two liquids. Surfactants are usually organic compounds that are amphipathic, meaning they contain both hydrophobic groups (their "tails") and hydrophilic groups (their "heads"). Therefore, they are soluble in both organic solvents and water. A surfactant can be classified by the presence of formally charged groups in its head. A nonionic surfactant has no charge groups in its head. The head of an ionic surfactant carries a net charge. If the charge is negative, the surfactant is more specifically called anionic; if the charge is positive, it is called cationic. If a surfactant contains a head with two oppositely charged groups, it is termed zwitterionic.

As used herein, ionic surfactants include anionic surfactants (based on sulfate, sulfonate or carboxylate anions), such as sodium dodecyl sulfate (SDS), ammonium lauryl sulfate, and other alkyl sulfate salts, sodium laureth sulfate (also known as sodium lauryl ether sulfate (SLES)), alkyl benzene sulfonate, soaps and fatty acid salts; cationic surfactants (based on quaternary ammonium cations), such as cetyl trimethylammonium bromide (CTAB) and other alkyltrimethylammonium salts, cetylpyridinium chloride (CPC), polyethoxylated tallow amine (POEA), benzalkonium chloride (BAC), benzethonium chloride (BZT); and zwitterionic (amphoteric) surfactants, such as dodecyl betaine, dodecyl dimethylamine oxide, cocamidopropyl betaine, coco ampho glycinate. As used herein, nonionic surfactants include alkyl poly(ethylene oxide), copolymers of poly(ethylene oxide) and poly(propylene oxide) (commercially called Poloxamers or Poloxamines), alkyl polyglucosides, fatty alcohols, cocamide MEA, cocamide DEA and cocamide TEA. Preferred surfactants include polysorbates (such as Tween) and Poloxamers (such as Pluronics).

Preferred anti-oxidative agents (antioxidants) include TPGS due to surfactant properties, BHA, BHT, t-butyl hydroquinone, calcium ascorbate, gallic acid, hydroquinone, maltol, octyl gallate, sodium bisulfite, sodium metabisulfite, tocopherol and derivates thereof, citric acid, tartaric acid, and ascorbic acid. Other antioxidants include trivalent phosphorous like e.g phosphite, phenolic antioxidants, hydroxylamines, lactones such as substituted benzofuranones. Hindered phenols, thiosynergists and/or hindered amines are useful for the long-term stability for liposomes, whereas the following antioxidants are suitable for use also in situation where the at least one proteinaceous substance is subject to oxidation: adds (ascorbic acid, erythorbic acid, etidronic acid, gallic acid, hypophosphorous acid, nordihydroguairetic acid, propionic acid etc.), phenols (e.g. BHA, BHT, t-butyl hydroquinone, dodecyl gallate, octyl gallate, 1,3,5-trihydroxybenzene), organic and inorganic salts (calcium ascorbate, sodium ascorbate, sodium bisulphite, sodium metabisulfite, sodium sulfite, potassium bisulphite, potassium metabisulphite), esteres (calcium ascorbate, dilauryl thiodipropionate, dimyristyl thiodipropionate, distearyl thiodipropionate), pyranon (maltol), and vitamin E (tocopherol, D-[alpha]-tocopherol, DL-[alpha]-tocopherol, tocopheryl acetate, d-[alpha]-tocopheryl acetate, dl-[alpha]-tocopheryl acetate. However, other anti-oxidative agents known in the art may be used according to the present invention.

Cyclodextrins (sometimes called cycloamyloses) make up a family of cyclic oligosaccharides, composed of 5 or more α-D-glucopyranoside units linked 1->4, as in amylose (a fragment of starch). Preferred cyclodextrins contain a number of glucose monomers ranging from six to eight units in a ring, creating a cone shape, thus denoting: α-cyclodextrin (six sugar ring molecule), β-cyclodextrin (seven sugar ring molecule) and γ-cyclodextrin (eight sugar ring molecule).

Furthermore, a certain number of additives may be added to the inventive composition in order to optimise its chemical, physical and mechanical properties in order to adapt it for the intended use. These additives include *inter alia* sodium benzoate, fatty acid esters or salts, trisodium phosphate, liquid paraffin, zinc oxide, calcium stearate, zinc stearate. The inventive composition may also contain other excipients as well, e.g. in order to improve the technical properties of the VPG composition so that it may be easier to produce or in order to improve the stability of the gel matrix. Excipients to stabilize the VPG matrix include polymers like spider silk proteins, chitosan and acrylate. A suitable pharmaceutically acceptable excipient for use in a composition of the invention may be selected from the group consisting of fillers, diluents, disintegrants, glidants, pH-adjusting agents, viscosity adjusting agents, solubility increasing or decreasing agents, osmotically active agents and solvents.

In a second aspect, the present invention relates to a method for the production of a pharmaceutical composition, comprising
(i) providing a preparation comprising a phospholipid and at least one proteinaceous substance, wherein the at least one proteinaceous substance is a biologically active protein, peptide or polypeptide;
(ii) homogenizing the preparation of (i) in a dual asymmetric centrifuge.

Contrary to the state of the art, the inventive method can be performed in a very simple and rapid way and enables also persons lacking specific know-how to produce the inventive composition. Due to the rapid practicability of the method, sensitive proteinaceous substances (e.g., sensible to degradation) may be enclosed in liposomes. Since the manufacturing proceeds sterile and very fast, the inventive composition may be used for the manufacture of pharmaceuticals, e.g., of injections, also and especially directly prior to the administration to patients ("bedside preparation").

Hence, the pharmaceutical composition according to the present invention is obtainable by a process comprising
(i) providing a preparation comprising at least one phospholipid and at least one proteinaceous substance, wherein the at least one proteinaceous substance is a biologically active protein, peptide or polypeptide;
(ii) homogenizing the preparation of (i) in a dual asymmetric centrifuge.

A "dual asymmetric centrifuge" (DAC) is a centrifuge where in addition to the processes of a classic centrifugation the vessel containing the material to be centrifuged is preferably rotated opposite to the direction of rotation, e.g., with a fourth to a third of the centrifuge speed. This results in a constant mixing of the material introduced into the DAC. The high centripetal force also enables viscous masses to be mixed (see EP 1 293 245). In this process a strong inner friction is generated in particular in viscous masses. Usually, the DAC is used for mixing pastes with pastes, pastes with powders, powders with powders etc. Typical application areas are the mixing of sealants and coating agents (such as, e.g., silicones, polyurethanes and acrylates), lacquers, inks and pigments and the mixing of one-, two- or multi-component products in liquid or paste-like forms. Within the context of the present invention, "dual asymmetric centrifuge" means the use of a Speedmixer® or another DAC, preferably a type DAC 150 FVZ Speedmixer® of the company Hausschild, Hamm, Germany, having a counter-rotation ratio of approximately 2.8:1 or 4.2:1, or a DAC having a similar counter-rotation ratio.

The method according to the invention may be performed as follows: the at least one phospholipid (and optionally further lipids and adjuvants) contemplated for the respective preparation and optionally one or several proteinaceous substance(s) are added to a suitable vessel (mixing vessel) in the dry state, subsequently the aqueous component optionally containing one or several proteinaceous substance(s) and further adjuvants is added, and the mixture is homogenized in a DAC. A corresponding (lipid) mixture optionally containing one or several proteinaceous substance(s) present as a solid solution may directly be employed as lipid component. Alternatively, the addition of the aqueous component may be performed in a metered manner during homogenization in the DAC. Also charging the aqueous component and adding the lipid component, optionally in a suited solvent, is possible.

Within the context of the invention, the "aqueous component" is water or an aqueous alcoholic and/or buffer-containing solution. In addition, salts and other low-molecular water-soluble substances may be dissolved therein.

In the inventive method, batch sizes of few milligrams up to 3,000g are easily possible with batch sizes of from 5 mg to 150g, particularly preferred of from 20 mg to 40 g, being preferred, larger batch sizes are technically also very well feasible. The method according to the invention is preferably performed using a DAC suited for taking-up reaction vessels having a size of from 0.1 to 110 ml, preferably of from 0.1 to 25 ml, particularly preferred of from 0.5 to 10 ml, and most preferred of from 0.5 to 2 ml. In the inventive method, a centrifugal force of at least 1.2 g, preferably at least 80 g, particularly preferred at least 300 g, and most preferred at least 550 to 1,000 g or from 620 to 1,500 g is applied on the substances to be homogenized. However, the maximum centrifugal force may be up to 3,000 g, preferably up to 2,500 g. In a preferred aspect, the homogenization in the production process is performed at a centrifugation speed of at least 2,000 rpm, with a speed of 3,500 rpm being particularly preferred.

If lipid mixtures comprising a phospholipid and one or more further lipids are used, said mixtures may be employed as single components or ready-mixed lipid mixtures ("solid solutions", that is, mixed crystals made from the separate lipids) in the inventive method. Due to the high inner friction in the homogenization process and the strongly increased contact events between the particles being formed and the proteinaceous substances to be enclosed, liposomes loaded with proteinaceous substances are formed in a short time. If the amount of the added aqueous component is kept low (resulting in relatively high lipid concentrations), a viscous lipid paste consisting of nanoparticles is formed, said paste being known, e.g., from WO 96/005808 for liposome gels. In specific cases, this approach is advantageous in that due to the high lipid concentration and hence a strong inner friction-small particles are formed especially efficiently and in the case of liposomes an especially high enclosure efficiency for water-soluble substances may be achieved (high amount of enclosed aqueous phase). Optionally, subsequently to the vesicle formation non-enclosed proteinaceous substance may be separated off.

The overall centrifugation time is from 30 s to 90 min. In preferred embodiments of the invention, the method further comprises controlling and/or adjusting the temperature of the preparation during the centrifugation process. This may be performed as follows: After charging a suitable vessel (mixing vessel) with the provided preparation comprising at least one phospholipid and at least one proteinaceous substance, the preparation is homogenized in a DAC at a centrifugation speed of at least 2,000 rpm for at least 5 minutes. In order to avoid heating of the preparation due to homogenization, the preparation is then incubated at a temperature of less than 10°C for at least 5 minutes and, subsequently, the procedure is repeated until an overall centrifugation time of at least 30 minutes, preferably 60 minutes, is reached. Controlling and/or adjusting the temperature of the preparation (incubation at a temperature of less than 10°C) may directly be employed in the centrifuge, for instance by reducing the centrifugation speed (or stopping the DAC) and cooling down the mixing vessel inside the centrifuge. Alternatively, the centrifuge may be cooled during homogenization. Suitable dual asymmetric centrifuges having a temperature control unit for adjusting the temperature inside the centrifuge are commonly known in the art. Alternatively, the mixing vessel may be removed from the DAC after interrupting the homogenization, which may then be incubated at the desired temperature (e.g. on crushed ice). Accordingly, this process of production has the advantage that it does not unnecessarily strain the proteinaceous substance which is critical with expensive and sensitive substances such as biologically active proteins, peptides and polypeptides. Surprisingly, it has been found that the encapsulation of proteinaceous substances in VPGs has no detrimental effects on the protein stability.

The method of the invention further allows a simple sterile working: only the filling of the mixture components into sterile vessels has to be performed under sterile conditions. After sealing the vessels, homogenization may be performed in nonsterile rooms.

In preferred embodiments of the present invention, homogenizing the preparation is performed in a dual asymmetric centrifuge without using any homogenization aid such as glass beads. It was known in the art that in order to improve homogenization and to minimize the mean particle size of the liposomes, homogenization aids (synonymous terms: dispersing aid, mixing aid) should be provided within the vessel or within containers arranged therein. Said homogenization aids may be a defined roughness within the inner wall of the vessel/the container; beads (made of glass or any other suitable material) in various amounts or sizes; blades on the inner vessel wall; or a suitably formed central and/or circumferential vessel insert. However, using homogenization aids has the drawback that a large percentage of the expensive and valuable biological substances to be encapsulated may stick to said homogenization aids, thereby avoid unnecessary loss of proteinaceous substance during homogenization, thus increasing the encapsulation efficiency. Accordingly, the scalable method for encapsulation of the at least one proteinaceous substance disclosed herein for the preparation and loading of vesicular phospholipid gels proved to be especially advantageous because of its high efficiency, while at the same time having extremely mild process conditions.

In a further specific embodiment of the invention, a method for the purpose of an "ad hoc" or an "ex tempore" production of the inventive compositions is provided. This embodiment is characterized by the production of the inventive compositions by means of a container of any format, size and material that is pre-filled with at least one phospholipid in an amount that is necessary to produce a VPG. A solution of the proteinaceous substance is separately provided in a suitable amount and concentration. The process is further characterized in that it encompasses the mixing of the at least one phospholipid in the container with the proteinaceous substance solution by means of an asymmetric centrifugation shortly before the resulting gel shall be used for its medical or pharmaceutical purpose. Such mixing would then directly lead to the inventive compositions according to the invention.

In a preferred embodiment the container with the at least one phospholipid and the container with the proteinaceous substance solution are tightly connected to each other by means of fittings as used for aseptic connections for medical use, e.g. LUER lock fitting and the like. In a further preferred embodiment of the invention both containers and their content are already sterile or have low bioburden and their contents are subsequently admixed into a sterile product or a product with low bioburden. In a further specific embodiment of the invention such a container (after mixing and preparation of a VPG) allows the application of the resulting product through a particularly designed application opening in the form of a syringe tip, a needle, a catheder or other similar helpful orifice and device variants. In a specific embodiment the container is flexible or has a movable piston-like closure on one or more sides or any other designs of similar functionality, allowing to press or squeeze out the inventive composition through the application opening.

In a further specific embodiment the container allows mixing at least two proteinaceous substance solutions having different protein contents with the prefilled phospholipid, the container combination thereby providing a standard VPG forming kit or part of a kit. This specific embodiment of the invention may be available in the form of prefilled phsopholipid containers of different sizes, preferable in such sizes, that the content after admixing the protein solution could be used for one patient for a specific application site, application type, type of surgery, type of wound, body opening etc. This embodiment can also be designed in a way that the inventive composition can be pressed into or sucked into a syringe-type device immediately after preparation, the device having been attached to the prefilled phospholipid container via a luer-lock or similar connection as described before. By the means of such a syringe filled with the inventive composition, the composition may then be directly applied to the patient or other point of use. This specific embodiment is especially useful for unstable proteins that may not be kept together with the phospholipid of the composition for a long storage period. It may also be preferable for the use of compositions when different drug concentrations are needed for different patients and where it would therefore be nearly impossible to provide the right dose for every patient by prefilled industrial products. The specific embodiment is also very useful in cases where the proteinaceous substance loaded VPGs are not used regularly and would expire after storage. The prefilled lipid container can be stored for a very long time, even frozen storage may be considered. It is to be understood that the description shall not be limited to any specific solutions of the basic concept described above but shall include every similar solution with the same or well related purpose to provide a method for an "ad hoc" preparation of the inventive compositions.

In a further aspect, the present invention relates to the inventive pharmaceutical composition for use in immunotherapy, comprising administering to a subject in need thereof, a therapeutically effective amount of the pharmaceutical composition, wherein the immunotherapy comprises (i) treating malignant cell growth by stimulating the immune system or (ii) treating an autoimmune disease or preventing rejection of transplanted organs or cells by reducing the normal immune response.

"Immunotherapy", as used herein, refers to treatment strategies based upon the concept of modulating the immune system to achieve a prophylactic and/or therapeutic goal. Many such strategies are commonly known in the art. For instance, "cancer immunotherapy" attempts to stimulate the immune system to reject and destroy tumors. Vice versa, "immune suppression therapy" dampens an abnormal immune response in autoimmune diseases or reduces a normal immune response e.g. to prevent rejection of transplanted organs or cells. Such immune tolerance therapies seek to reset the immune system so that the body stops mistakenly attacking its own organs or cells in autoimmune disease or accepts foreign tissue in organ transplantation. A brief treatment may reduce or eliminate the need for life-long immunosuppression and the chances of attendant side effects, in the case of transplantation, or preserve the body's own function, at least in part, in cases of autoimmune disorders.

Therapeutic antibodies suitable for immunotherapy include the following antibodies:

**Table 1: Approved therapeutic antibodies**

| **Antibody** | **Brand name** | **Approved treatment(s)** |
|---|---|---|
| Abciximab | ReoPro | Cardiovascular disease |
| Adalimumab | Humira | Inflammatory diseases (mostly auto-immune disorders) |
| Alemtuzumab | Campath | Chronic lymphocytic leukemia |
| Basiliximab | Simulect | Transplant rejection |
| Bevacizumab | Avastin | Colorectal cancer |
| Cetuximab | Erbitux | Colorectal cancer |
| Daclizumab | Zenapax | Transplant rejection |
| Eculizumab | Soliris | Inflammatory diseases including paroxysmal nocturnal hemoglobinuria |
| Efalizumab | Raptiva | Inflammatory diseases (psoriasis) |
| Ibritumomab tiuxetan | Zevalin | Non-Hodgkin lymphoma (with yttrium-90 or indium-111) |
| Infliximab | Remicade | Inflammatory diseases (mostly auto-immune disorders) |
| Muromonab-CD3 | Orthoclone OKT3 | Transplant rejection |
| Natalizumab | Tysabri | Inflammatory diseases (mainly autoimmune-related multiple sclerosis therapy) |
| Omalizumab | Xolair | Inflammatory diseases (mainly allergy-related asthma therapy) |
| Palivizumab | Synagis | Viral infection (especially Respiratory Syncytial Virus (RSV) |
| Panitumumab | Vectibix | Colorectal cancer |
| Ranibizumab | Lucentis | Macular degeneration |
| Gemtuzumab ozogamicin | Mylotarg | Acute myelogenous leukemia (with calicheamicin) |
| Rituximab | Rituxan, Mabthera | Non-Hodgkin lymphoma |
| Tositumomab | Bexxar | Non-Hodgkin lymphoma |
| Trastuzumab | Herceptin | Breast cancer |

However, any therapeutic antibody suitable for immunotherapy and known to the skilled person may be encapsulated within the inventive compositions.

Dosages of approved therapeutic antibodies suitable for the desired therapy are generally chosen in accordance with a number of factors, e.g., the age, size and general condition of the patient and the medical condition being treated, and determined by a variety of means, for example, dose ranging trials, well known to, and readily practiced by persons of ordinary skill in art given the teachings of this invention. For immunotherapy, the inventive composition comprising at least one proteinaceous substance (e.g., one of the above-listed approved antibodies) suitable for the desired therapy can be administered to a subject by any method that stimulates the aforesaid immune response or reduces the normal immune response.

Generally, the exact administration method is determined by the particular composition to be administered and is readily known to the skilled person. For instance, for parenteral administration by injection, the injection can be *in situ* (i.e., to a particular tissue or location on a tissue, e.g., into a tumor or into inflamed tissue), intramuscular, intraperitoneal, intrasynovially, intraarticularly (into a bone joint, e.g. knee joint) or by another parenteral route. For example, the inventive composition may be administered by subcutaneous or intradermal injection. In some cases other routes can be used, e.g. intraperitoneal injection, or *in situ* injection into target tissue. The compositions according to the invention may further be administered by intramuscular injection. The compositions of the invention can also be administered by a non-parenteral route, e.g, by buccal, urethral, vaginal, or rectal administration.

Preferably, the inventive compositions are administered to a particular tissue or location on a tissue ("local depot for local delivery"), thus avoiding possible drawbacks of a systemic delivery (such as possible undesired side effects of the proteinaceous substance or the necessity to use large doses of the proteinaceous substance to achieve a pharmaceutically active amount of the substance at the site of action).

The invention also provides that the sustained release composition may contain one or more immunopotentiating agents (for example, the composition may contain either an interleukin or an interferon or both). The "immunopotentiating agent", as used herein, is a proteinaceous substance that enhances responsiveness of the subjects' immune system to a tumor or pathogenic organism, such as a virus, present in the subject. Included in this category of immunopotentiating agents are a number of cytokines classified as "interleukins". These include, for example, interleukins 1 through 12. Also included in this category, although not necessarily working according to the same mechanisms, are interferons, and in particular gamma interferon (γ-IFN), tumor necrosis factor (TNF) and granulocyte-macrophage-colony stimulating factor (GM-CSF). Hence, depending on the site and mode of administration of the composition, the liposomes of the VPG according to the invention may contain a specific cytokine. For example, when administration is directly at the site of the tumor, a cytokine which directly kills cells, such as TNF, may be most effective. Alternatively, when the administration is at a distant site, a cytokine such as GM-CSF, or other molecule which can generally recruit immune cells, such as T-cells, granulocytes and macrophages, may be most effective. Those of skill in the art will be able to ascertain which immunopotentiating agent to use, depending on which biological function is desired.

In a further aspect, the present invention thus relates to the inventive pharmaceutical composition for use in stimulating selective tissue regeneration in the treatment of tissue defects, e.g. in the course of surgical interventions, comprising administering to a subject in need thereof, a therapeutically effective amount of the pharmaceutical composition.

In the context of the present invention, a "subject" can be a mammal, such as a human, in particular a human patient in need of treatment, a companion animal, or a farm animal.

Accordingly, the term "tissue" is used for mammalian tissue and is preferably selected from the group consisting of joints, muscle tissue, connective tissue, tendons, organs and skin. In various configurations, the tissue can be bone, cartilage, tendon tissue, ligament tissue, soft tissue such as vascular tissue, dermal tissue or muscle tissue, or a combination thereof. In some other configurations, a site in need of tissue growth can comprise tendon tissue, ligament tissue, vascular tissue, dermal tissue, periodontal tissue, intervertebral disc tissue, hyaline cartilage, fibrous cartilage, elastic cartilage, a nerve tunnel or a combination thereof. However, in the context of the present invention, "mammalian tissue" is in general any tissue having an extracellular matrix that can be isolated from the mammal and decellularized. The tissue can thus be any mammalian tissue, including small intestine, large intestine, stomach, lung, liver, kidney, pancreas, placenta, heart, bladder, prostate, tissue surrounding growing enamel, tissue surrounding growing bone, body orifices and any fetal tissue from any mammalian organ.

Accordingly, a mammalian recipient of a composition of the present teachings can be a human patient in need of treatment, such as an individual having an injury, a surgery (suffering from surgical defects in the course of surgical interventions) or degenerative disease of bone, cartilage, tendon tissue, ligament tissue, vascular tissue, dermal tissue, muscle tissue or a combination thereof. A skilled artisan such as a surgeon or a dermatologist can implant or inject the inventive composition at a site within the body of the patient. The introduced composition can then release the proteinaceous substance, thereby accelerating or promoting the healing of adjacent tissue.

In further embodiments, a human in need of treatment can be a patient having muscle atrophy or dystrophy resulting from a disease or disorder such as AIDS, diabetes, muscular dystrophy or cancer.

In yet another example, a human in need of treatment can be a patient recovering from a traumatic injury to an internal organ which requires revascularization. In such an individual, growth of new blood vasculature can be directed and/or promoted by surgical implantation or injection of a composition according to the invention.

In various aspects, administering the inventive composition can comprise surgical implantation or injection at a site in need of tissue growth or repair, or a combination of surgical implantation and injection of the composition. Promoting tissue growth or repair can include conducting tissue growth, or inducing tissue growth, or a combination thereof. In various aspects, a site in need of tissue growth can comprise tendon tissue, ligament tissue, vascular tissue, dermal tissue, periodontal tissue such as a periodontal ligament, intervertebral disc tissue, hyaline cartilage, fibrous cartilage, elastic cartilage, a nerve tunnel or a combination thereof.

In various aspects, the methods include administration of a pharmaceutical composition described herein by surgical implantation and/or injection into or adjacent to the muscle tissue of a patient. These methods can thus be used, in some aspects, to stimulate muscle growth or repair in an individual patient in need, such as a person experiencing loss of muscle mass resulting from a disease having symptoms of dystrophic or atrophic muscle, such as, without limitation, AIDS, muscular dystrophy, diabetes or cancer. The composition may also be used to fill a tumor excision site after excision of the tumor, promoting wound healing at the site and tissue regeneration of the lost tissue.

In various aspects, methods of administering to a subject a pharmaceutical composition described herein can include injecting the matrix and/or implanting the matrix into the subject. In various configurations, a site in need of tissue growth can comprise dermis, a rotator cuff tendon, an Achilles tendon, a ligament such as an anterior cruciate ligament (ACL), a posterior cruciate ligament, (PCL), a medial collateral ligament, a lateral collateral ligament or a periodontal ligament, a sphincter such as an anal sphincter, a urethral sphincter, an esophageal sphincter or an antral sphincter, herniated tissue such as an abdominal hernia, a Cooper's hernia, a diaphragmatic hernia, an epigastric hernia, a femoral hernia, an incisional hernia, an inguinal hernia, an intervertebral disc hernia, a Littre's hernia, an obturator hernia, a pantaloon hernia, a perineal hernia, a preperitoneal hernia, a Richter's hernia, a sciatic hernia, a sliding hernia, a Spigelian hernia or an umbilical hernia, an intervertebral disc nucleus, an intervertebral disc annulus, periosteal tissue, neural tissue such as central nervous system tissue (including spinal cord tissue) and demyelinated neural tissue, a nerve tunnel such as a nerve tunnel traversing bone tissue, a mitral valve, a tricuspid valve, an aortic heart valve, a pulmonary heart valve, vascular tissue comprising a stent, stenotic cardiovascular tissue, costal cartilage, meniscus cartilage, epiglottic cartilage, laryngeal cartilage such as arytenoid cartilage, cricoid cartilage, cuneiform cartilage and corniculate cartilage, external ear cartilage, or auditory tube cartilage.

In various aspects, the present invention further relates to a pharmaceutical composition according to the invention for stimulating selective tissue regeneration in the treatment of surgical defects in the course of surgical interventions, comprising administering to a subject in need thereof, a therapeutically effective amount of the pharmaceutical composition.

For stimulation of selective tissue regeneration, the inventive composition comprises at least one proteinaceous substance suitable for the desired therapeutic effect (e.g. the treatment of surgical tissue defects in the course of surgical interventions). The proteinaceous substance can be, for example, a growth factor, or any other type or protein that might stimulate some part of the tissue regenerative, wound healing, or new tissue generating process, including a collagen, a proteoglycan, a glycosaminoglycan (GAG) chain, a glycoprotein, a growth factor, a cytokine, a cell-surface associated protein, a cell adhesion molecule (CAM), an angiogenic growth factor, an endothelial ligand, a matrikine, a matrix metalloprotease, a cadherin, an immunoglobin, a fibril collagen, a non-fibrillar collagen, a basement membrane collagen, a multiplexin, a small-leucine rich proteoglycan, decorin, biglycan, a fibromodulin, keratocan, lumican, epiphycan, a heparan sulfate proteoglycan, perlecan, agrin, testican, syndecan, glypican, serglycin, selectin, a lectican, aggrecan, versican, nuerocan, brevican, cytoplasmic domain-44 (CD-44), macrophage stimulating factor, amyloid precursor protein, heparin, chondroitin sulfate B (dermatan sulfate), chondroitin sulfate A, heparan sulfate, hyaluronic acid, fibronectin (Fn), tenascin, elastin, fibrillin, laminin, nidogen/entactin, fibulin I, fibulin II, integrin, a transmembrane molecule, platelet derived growth factor (PDGF), epidermal growth factor (EGF), transforming growth factor alpha (TGF-alpha), transforming growth factor beta (TGF-beta), fibroblast growth factor-2 (FGF-2) (also called basic fibroblast growth factor (bFGF)), thrombospondin, osteopontin, angiotensin converting enzyme (ACE), or a vascular epithelial growth factor (VEGF).

"Therapeutically effective amount" is a term meant to capture the idea that you need to apply enough of the composition in sufficient strength so that the composition can have a positive effect on the tissue that is being treated in the subject. That the amount is therapeutically effective is determined by the composition's ability to have a regenerative or wound healing effect at the site where the composition contacts the tissue. A therapeutically effective amount is determinable by routine testing in patients with tissue defects. In general a minimal therapeutically effective amount would be considered sufficient composition to contact amply all of the defect in the tissue, or otherwise act at the site to regenerate tissue, or generate new tissue.

Regenerating tissue at the defect can be one response elicited from the step of placing the extracellular matrix composition in contact with the defect. If the defect is a wound in need of healing, wound healing may be another response that occurs as a result of placing the extracellular matrix at the wound site. Further, the biological activity might be characterized as generating new tissue at a site. In general any term that identifies that the tissue could benefit from a healing or tissue regeneration fits within the scope of the use for the composition. Thus regenerating tissue, healing a wound, or generating new tissue are three such phrases to describe the biological process that is hoped for, but the not the only phrases that can be used to describe the effects achieved when the composition is placed in the mammal at a site of defect or damage in tissue.

For stimulating selective tissue regeneration in the treatment of tissue defects, the inventive composition comprising at least one proteinaceous substance (e.g., one or more of the above-listed growth factors or cytokines) can be administered to a subject by any suitable method. The exact method selected is determined by the particular therapy and is readily known to the skilled person. In preferred embodiments of the invention, administering the pharmaceutical composition comprises injection, needle-free injection, vaginal application, nasal application, urethral application, topical application, peroral application or surgery.

In still another embodiment, the present invention relates to a pharmaceutical composition according to the invention for treating a condition associated with abnormal insulin secretion, comprising administering to a subject in need thereof, a therapeutically effective amount of the pharmaceutical composition comprising insulin or glucagon as the at least one proteinaceous substance. Conditions associated with abnormal insulin secretion include insulin resistance syndrome, pre-diabetic conditions, metabolic syndrome, type 1 and type 2 diabetes, cardiac disease, diabetes-associated vascular disease, atherosclerosis, hypertension, diabetes-associated lipid metabolism disorders, obesity, and neurodegenerative disorders.

In a further aspect, the present invention relates to the use of a pharmaceutical composition according to the present invention for the manufacture of a medicament for immunotherapy, comprising administering to a subject in need thereof, a therapeutically effective amount of the pharmaceutical composition, wherein the immunotherapy comprises (i) treating malignant cell growth by stimulating the immune system or (ii) treating an autoimmune disease or preventing rejection of transplanted organs or cells by reducing the normal immune response.

In a further aspect, the present invention relates to the use of a pharmaceutical composition according to the present invention for the manufacture of a medicament for stimulating selective tissue regeneration in the treatment of tissue defects, comprising administering to a subject in need thereof, a therapeutically effective amount of the pharmaceutical composition. In this case, the tissue is preferably selected from the group consisting of joints, muscle tissue, connective tissue, tendons, organs and skin.

In a specific embodiment, the inventive composition may be applied by heating the VPG directly before application up to a temperature that is well tolerable at the site of application at the human or animal body or other site of use in order to allow better and easier application due to better fluidity, syringability etc. Such heating can be applied by different means including microwaves and other wave-bound forms of energy transfer.

The inventive composition may be supplied with or in a specialized applicator. The device will have an outlet for the solution, an ejector for expelling the gel through the outlet and a hollow tube fitted to the outlet for inserting the gel into a site of the body such that the gel can be applied to the desired site. The applicator will be sterile, and preferably disposable. In one embodiment the applicator is a conventional syringe, with a length of tubing pre-connected to the outlet of the syringe, prefilled with the inventive composition. The inventive compositions can be packaged in a kit which contains a means for administering the composition, such as a syringe, and instructions for administering the composition.

The invention is further described by the figures and the following examples, which are solely for the purpose of illustrating specific embodiments of this invention, and are not to be construed as limiting the scope of the invention in any way.

### EXAMPLES

### Example 1:

### Preparation of Vesicular Phospholipid Gels (VPGs) by Asymmetric Centrifugation

In the following, a Speedmixer® of the type DAC 150 FVZ, Hausschild, Hamm, Germany having a counter-rotation ratio of about 4.2:1 has been used to provide the compositions exemplified herein. Unless stated otherwise, the method for the production of the inventive compositions is based on the preparation methods disclosed in EP 1 674 081 which were revised and improved as follows:

Directly after weighing of the constituents of the preparation (lipids, at least one proteinaceous substance and an aqueous compound), the homogenization was performed in the dual asymmetric centrifuge in multiples of 9 minutes.

However, proteinaceous substances such as proteins are known to adsorb on different kind of surfaces. In order to avoid protein loss due to adsorption on glass beads the preparation of the compositions was performed without using any homogenization aid such as glass beads (EP 1 674 081 teaches to use glass beads in order to facilitate the production of a liposome dispersion containing very small particle sizes). Unless stated otherwise, the compositions were prepared with two different kinds of phospholipids (PHOSPHOLIPON 85G and 90G) with concentrations ranging from 30 wt-% to 60 wt-%, respectively. The centrifugation time varied from 30 minutes to 60 minutes, at the same centrifugation speed, 3500 rpm. In order to avoid heating of the gels due to the homogenization, the centrifugation cycles were stopped after 9 min. The preparations were incubated at 2-8°C for 10 min and subsequently the procedure was repeated until the overall centrifugation (60 minutes, unless stated otherwise) was reached.

As a model drug for the proteinaceous substance, FITC-Dextran was used. The concentration of FITC-Dextran was ranging from 1.1 mg/g to 3.4 mg/g. The batch size was between 3.3 g and 5.0 g. Experiments were conducted as shown in the following Tables:

**Table 2A: Lipid concentration (Phospholipon 85G)**

| No. | 1 | 2 | 3 | 4 | 5 |
|---|---|---|---|---|---|
| Lipid Content /mg/g | 400 | 450 | 500 | 550 | 600 |

| Lipid Type | Phospholipon 85G | | | | |
|---|---|---|---|---|---|
| FITC concentration /mg/g | 2.2 | 2.2 | 2.2 | 2.2 | 2.2 |
| Centrifuge Time/min | 60 | 60 | 60 | 60 | 60 |

**Table 2B: Lipid concentration (Phospholipon 90G)**

| No. | 6 | 7 | 8 | 9 | 10 |
|---|---|---|---|---|---|
| Lipid Content /mg/g | 400 | 450 | 500 | 550 | 600 |

| Lipid Type | Phospholipon 90G | | | | |
|---|---|---|---|---|---|
| FITC concentration /mg/g | 2.2 | 2.2 | 2.2 | 2.2 | 2.2 |
| Centrifuge Time/min | 60 | 60 | 60 | 60 | 60 |

**Table 2C: FITC-Dextran concentration and centrifugation time**

| No. | 11 | 12 | 13 | 14 | 15 |
|---|---|---|---|---|---|
| Lipid Content /mg/g | 550 | 550 | 550 | 550 | 550 |

| Lipid Type | Phospholipon 90G | | | | |
|---|---|---|---|---|---|
| FITC concentration /mg/g | 1.1 | 3.4 | 2.2 | 2.2 | 2.2 |
| Centrifuge Time /min | 60 | 60 | 60 | 30 | 45 |

Although the production was performed without using any homogenization aid such as glass beads, it was surprisingly found that all obtained compositions appeared homogenous and, depending on the lipid content of the formulation, the viscosity of the obtained gels could be modified.

### Example 2:

### Rheology and Texture analysis

The rheology behavior of the inventive compositions was studied by a rotational viscometer. Test system: PP 50, rotation plate; Temperature: 25°C; Shearrate: 10-100 1/s. The viscosity data of different compositions at a shearrate of 37.9 1/s were used for evaluation. In addition to rheology, texture analysis was carried out. Compared to rheology measurements texture analysis is a less time consuming, simple way with less sample amount to predict the viscosity of the gels. Texture analysis (TA. XT plus, Stable Micro Systems, UK) of the compositions was performed with a microprobe of 4 mm in a gel volume of 1.5 ml. The test speed was set to 0.50 mm/s, and the test distance was 4.000 mm. The gel strength of the VPGs were measured and represented as the maximal force by the average of 3 parallels.

To evaluate the influence of different lipid types and different lipid concentrations, the compositions were prepared either with Phospholipon 85G or with Phospholipon 90G. The lipid concentration was varied from 40 to 60 wt-% lipid (400 to 600 mg/g VPG). All the formulations showed a semi-solid consistency except the formulation prepared with 40 wt-% Phospholipon 90G, which behaved more like a viscous liquid. Rheology tests, as well as texture analysis, were conducted to judge the viscosity of the VPGs.

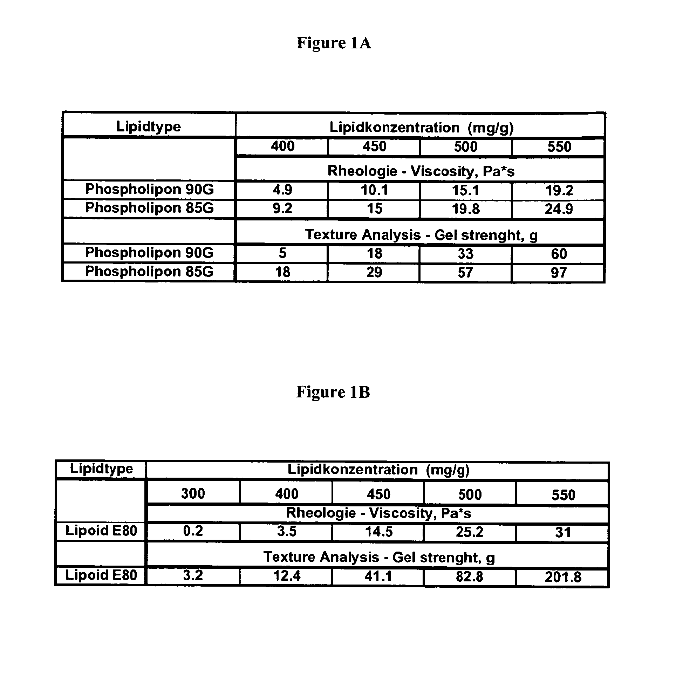

Figure 1A summarizes the viscosity as well as the maximal force of texture analysis increases with increasing lipid concentration. The viscosity data was compared at the shear rate of 37.9 1/s. For lipid concentrations from 40 to 55 wt-% (400 to 550 mg/g VPG) the viscosity ranged from 9.2 to 24.9 Pa·s for VPGs prepared with Phospholipon 85G and from 4.9 to 19.2 Pa·s for gels prepared with Phospholipon 90G. The maximal force of the compositions determined by texture analysis varied from 18 g to 97 g for Phospholipon 85G and from 5 g to 60 g for Phospholipon 90G with phospholipid concentration increasing from 40 to 55 wt-% (400 to 550 mg/g VPG).

In general, the viscosity and force of compositions prepared with Phospholipon 85G were greater than those prepared with Phospholipon 90G. At the phospholipid concentration of 55 wt-%, the viscosity was 24.9 Pa·s for VPGs prepared with Phospholipon 85G, and 19.2 Pa·s for those with Phospholipon 90G; as for maximal force, the data was 97 g for Phospholipon 85G and 60 g for Phospholipon 90G, respectively. The trend of maximal force of texture analysis was in accordance with the viscosity data from rheology tests.

Besides the evaluation considering Phosopholipon 85G and 90G as the phospholipid component, similar experiments were carried out with Lipoid E80. As can be seen in Figure 1B, the viscosities as well as the maximum force values increased when increasing the lipid concentration of lipoid E80 from 30% to 55% (300-550mg/g VPG). Without wishing to be bound by any theory, it is believed that a more rigid gel structure detected for gels with a higher lipid concentration is associated with slower release rates. Hence, it should be possible to achieve a desired release profile by optimizing the formulation of VPGs with respect to its viscosity.

### Example 3:

### Release of a macromolecular model drug

In order to evaluate the potential of the inventive compositions for the sustained release of proteins and other proteinaceous substances, the model drug FITC-Dextran (40kDa) was encapsulated in VPGs. For assessment of the *in vitro* release of drug from the inventive VPG-formulations a test method based on a custom-made flow-through cell, as reviewed by M. Brandl and U. Massing in "Liposomes - a practical approach", V.P. Torchilin and V. Weissing, Ed., 2nd edition (2003), was established.

An acceptor medium (buffered aqueous medium) is run through the cell at a rate of 1 ml/h (The flow rate is 10 ml/hr in the original method. However, the flow rate is reduced due to the detection need.) to mimic the flow of tissue fluid at the site of injection or implantation of the composition. Fractions collected over distinct time intervals were analyzed for the macromolecular model drug concentration by fluorescence photometry. Thereby, the lipids within the release fractions were dissolved by adding ethanol and the released FITC-dextran was subsequently quantified with fluorescence photometry (Ex: 488nm, Em: 517nm).

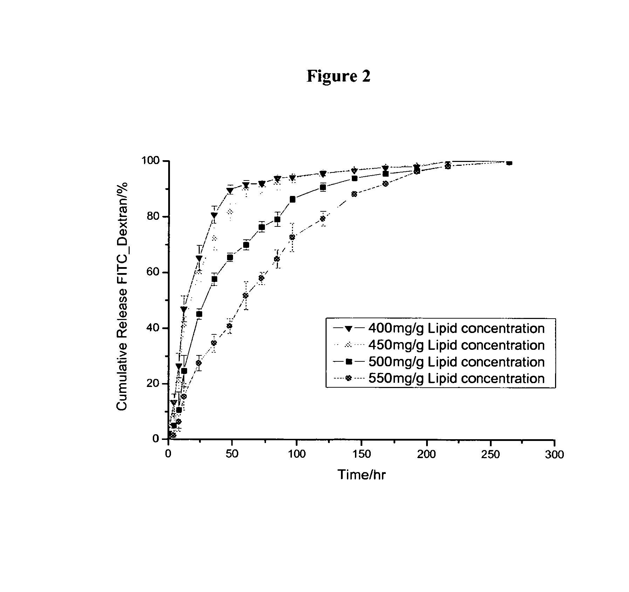

As can be seen in Figure 2, the release behavior could be adjusted by the variation of the phospholipid concentration. Slower release rates were observed with increasing the lipid concentrations. For instance, the release of compositions prepared with 55 wt-% Phopholipon 85G lasted more than 12 days, whereas the FITC-Dextran liberation was terminated after 4 days when the lipid concentration was 40 wt-%. This further confirmed that it should be possible to achieve a desired release profile for proteinaceous substances by optimizing the formulation of VPGs with respect to its viscosity.

### Example 4:

### Particle size analysis

An important morphological characteristic of VPGs is the size distribution. The size of the liposomes upon redispersions and release fractions were analyzed by Photo Correlation Spectroscopy (PCS). Due to the much too high concentration of vesicles, VPGs were diluted with excess PBS buffer (pH 7.4) by vortex and the resulting dispersions contained about 0.5 mg/ml lipids.

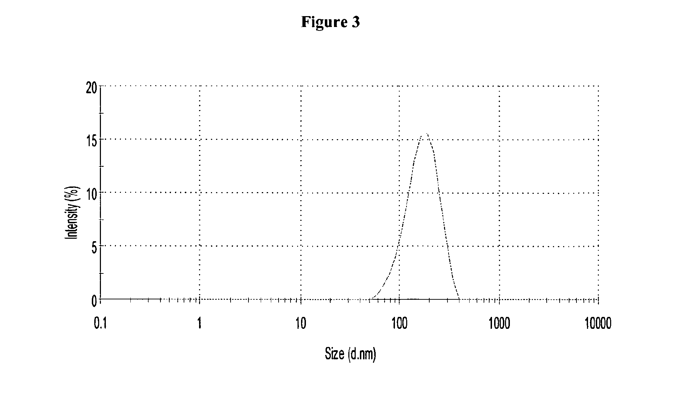

All the formulations showed the average diameter in the magnitude of 160 to 260 nm (by intensity) with a broad size distribution (Fig. 3). This indicates that the achievement of a certain particle size is of less importance for the sustained release of macromolecular drugs from homogeneous VPGs.

### Example 5:

### Effect of the manufacturing process on protein stability

Due to their fragile, three-dimensional macromolecular structure proteins are susceptible to a variety of chemical and physical degradation pathways which might account not only in the loss of the biological function but might also result in severe adverse reaction upon administration. Thus, the maintenance of the native protein structure is essential for the success of a certain protein formulation.

In order to evaluate whether the preparation process of the compositions has detrimental effects on the protein stability, non-reducing SDS-PAGE with subsequent silver staining was applied. This is a very sensitive method that allows the detection of protein aggregates as well as protein fragments. However, the lipid matrix interfered with the separation of the protein bands and several organic solvents and surfactants including chloroform, dichloromethane, ethanol, and triton were tried out to remove the lipids.

The VPGs comprised 500 mg/g lipids and 3.7 mg/g - 4.0 mg/g protein. 0.05g VPGs were redispersed in 1ml PBS buffer and organic solvents or detergents were added to disrupt the liposomes and extract the proteins. The aqueous phase was taken and loaded to the wells. Gel electrophoresis (SDS-PAGE) was conducted to evaluate the protein integrity in the formulation (non-reducing conditions were used with silver staining). The samples were diluted in a pH 6.8 Tris-buffer containing 2 % SDS, and incubated for 20 mins at 90 °C. 20µl was loaded into the gel wells (NuPAGE® Novex 10 % Bis-Tris Pre-Cast Gel 1.0 mm, Invitrogen, Groningen, Netherlands). Unstained molecular standard (Mark12 ^{™}, Invitrogen, Groningen, Netherlands) was used as markers. Electrophoresis was performed at a constant current mode of 40 mA in a MES running buffer (Invitrogen Groningen, Netherlands). The gels were stained with silver staining kit (SilverXpress® Stain Kit, Invitrogen), and dried using a DryEase ® Gel Drying System (Invitrogen).

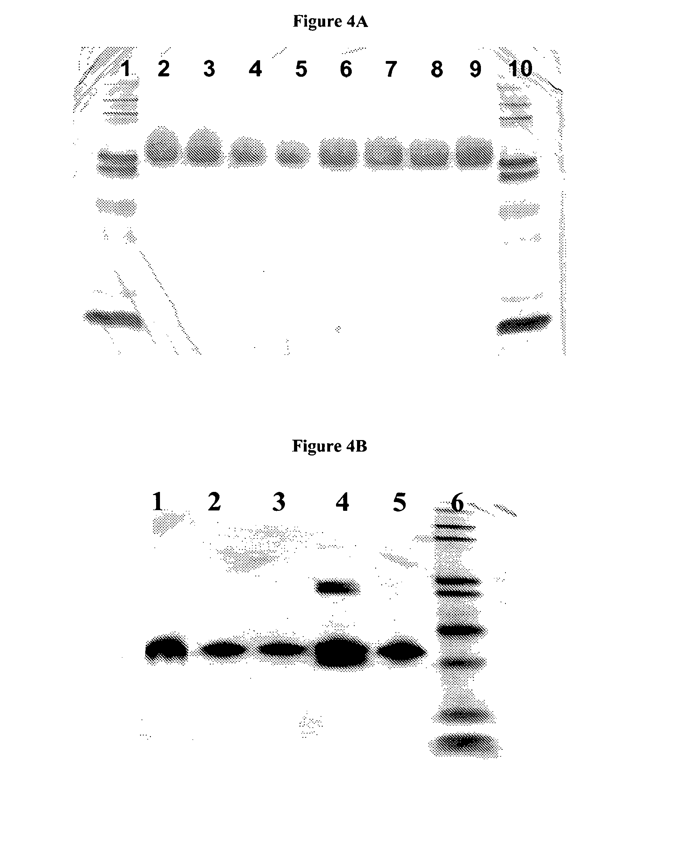

The extraction method using chloroform got the highest recovery of EPO. SDS-PAGE of erythropoietin (EPO) extracted from a freshly prepared VPG formulation is shown in Figure 4A. Importantly, no degradation bands are observed, which indicates that the preparation process did not affect protein stability.

In a second experiment, G-CSF was encapsulated into a similar VPG formulation. Compared to EPO G-CSF is a more hydrophobic protein, which accounts for a particular sensitivity of G-CSF to unfolding and aggregation. The choice of such a model protein with a highly pronounced instability should prove the general capability of the developed manufacturing method for the processing of pharmaceutical proteins.

Again, SDS-PAGE with subsequent silver staining was used as characterization method. In accordance to the analytics of EPO the phospholipid matrix was removed by the use of chloroform, dichloromethane, ethanol, or triton, respectively. As it can be gathered from Figure 4B, the dissolution of the phospholipids by triton resulted in the highest recovery of G-CSF without any detection of degradation products. Though the methods using chloroform and dichloromethane revealed lower extraction efficiencies, none of these methods featured aggregation or fragmentation of G-CSF. Only when ethanol was used as extraction solvent a higher molecular weight band was detected beside monomeric G-CSF. Without wishing to be bound by any theory, this indicates that the detected protein aggregation was a result of the extraction process and not of the inventive preparation process. Accordingly, both model proteins EPO and G-CSF suggest that the encapsulation of proteins in VPGs has no detrimental effects on the protein stability.

### Example 6:

### Protein release data

### 6.1. Erythropietin

For assessment of the *in vitro* release of drug from the inventive VPG-formulations a test method based on a custom-made flow-through cell, as reviewed by M. Brandl and U. Massing in "Liposomes - a practical approach", V.P. Torchilin and V. Weissing, Ed., 2nd edition (2003), was established. An acceptor medium (buffered aqueous medium) is run through the cell at a rate of 1ml/h, to mimic the flow of tissue fluid at the site of injection or implantation of the composition.

For the experiment set, the concentration of lipids (Egg PC, LIPOID E80) was varied between 300 and 550 mg/g at an EPO concentration of 3.8 mg/g. Homogenizing was carried out in a DAC for a total mixing time of 45mins. To quantify released proteinaceous substances fractions were collected over distinct time intervals and analyzed by reversed-phase HPLC (Spectra-Physics HPLC system (San Jose, CA, USA), equipped with a ternary gradient pump Spectra-Physics SP 8800, an autosampler Spectra Series AS 100 and a UV detector Spectra Series 1100. The system was controlled by HPLC ChromQuest software). The UV detector was operated at 215nm. The injection volume was 100µl.

Prior to the HPLC performance, an extraction method was needed to separate the protein from lipids within the release fractions. Different organic solvents and surfactants were tried out to extract protein. The extraction method should result in good recovery of the protein amount, and moreover, have no (or at least marginal) influence on the protein stability. Chloroform was evaluated and considered suitable for EPO extraction.

Gradient elution was needed to quantify released EPO with a Phenomenex C18 Jupiter column (5 µm, 250 x 4.6 mm). Two eluents of the solvent system were: (A) 0.1% TFA and (B) 84% CH₃CN, 0.08% TFA. The gradient started with 80% eluent A, over a period of 10min decreased to 40% A and then lasted for 5min. Afterwards, eluent A decreased to 0% in 5 min, and finally returned to 80% over a 5min period. The flow-rate was 0.5 ml min⁻¹. The retention time of EPO was 15-16 min. Three independent measurements per sample were performed.

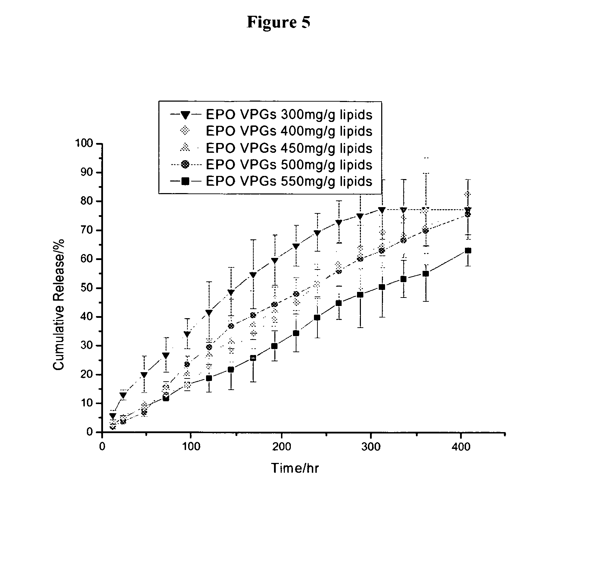

It can be seen in Figure 5 that increasing the phospholipid concentration from 300 mg/g to 450 mg/g to 550 mg/g decreases the release rate of EPO from the compositions. The release tests lasted over 400hrs. Surprisingly, minor various in the phospholipid contents between 400 and 500 mg/g did not show a significant influence on the in-vitro release data.

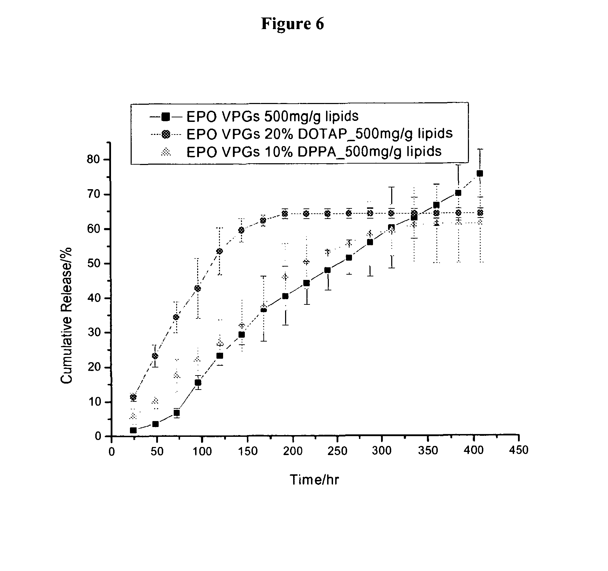

In order to investigate the influence of the phospholipid charge and thus of possible ionic interactions between the EPO and the phopsholipid vesicles, two VPGs formulations with either cationic (lipoid E 80 and 1,2-dioleoyloxy-3-trimethylammonium propane chloride (DOTAP) in a ratio of 8:2) or anionic (lipoid E 80 and dipalmitoylphosphatidic acid (LIPOID DPPA) in a ratio of 9:1) vesicles were investigated, respectively. For both formulations the total lipid concentration was 500 mg/g. As shown in Figure 6, DPPA-loaded VPGs showed a comparable release profile to neutral VPGs, while DOTAP-loaded cationic VPGs revealed a far increasing release rate, which reached already 76% after 150 hours.

### 6.2. Granulocyte-colony-stimulating factor (G-CSF)

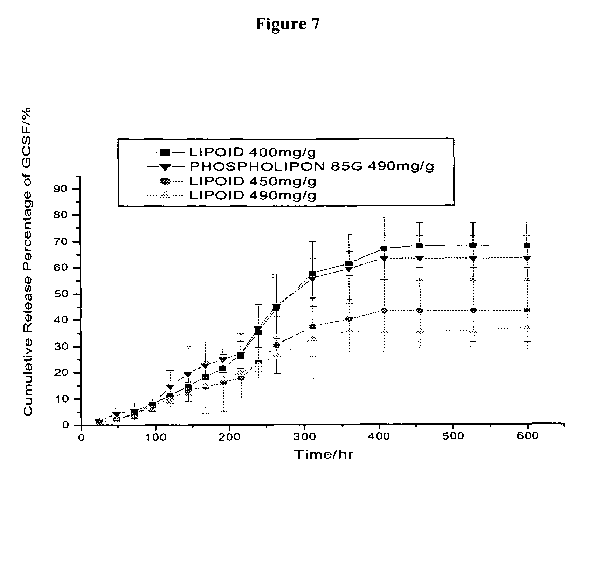

As shown in Figure 7, a reduction of the lipid concentration from 450 or 490 mg/g to 400 mg/g resulted in a drastic increase in the total amount of delivered G-CSF. While gels prepared with 450 or 490 mg/ml delivered only 37 or 43% of incorporated G-CSF, 69% of the protein were released when only 400 mg/g phospholipid were present. The release profiles of VPGs containing different lipid concentrations were found nearly identical until 200 hours, afterwards an increase in the release rates was observed for gels containing 400 mg/ml phsopholipid, whereas the release rates for gels with 450 and 490 mg/g lipid levelled off.

Interestingly, compositions prepared with 490 mg/g Phospholipon 85G showed the same release kinetics than gels prepared with 400 mg/ml Lipoid E80.

Without wishing to be bound by any theory, these observation might be explained by the fact that the same lipid concentration of Phospholipon 85G and Lipoid E80 resulted in less rigid gels in the case of Phospholipon 85G. Accordingly, a less rigid gel structure might result in an accelerate erosion of the gel and, in consequence, a faster release rate. This further confirmed that it should be possible to achieve a desired release profile for proteinaceous substances by optimizing the formulation of VPGs with respect to its viscosity.

Surprisingly, both model proteins were found to be delivered from the VPG compositions in linear manner. The release of EPO lasted about 400 hours with an almost zero-order kinetics. G-SCF was liberated from the gels with a slightly lower rate constantly over 375 hours. With respect to the later use *in vivo* such release profiles are especially advantageous since constant blood or tissue levels without any over- or underdose are achievable over prolonged periods of time. Furthermore, both kinetics lack a burst effect, which is mostly undesired since initial high release rates from sustained release systems may lead to drug concentrations near or above the toxic level *in vivo.* Furthermore, the drug released during burst might be metabolized and excreted without being effectively utilized.

### Example 7:

### Stability of Erythropoietin within VPGs

In order to investigate the stability of EPO within the VPG formulations a gel consisting of 500 mg/g phospholipid and 4.0 mg/g EPO was prepared according to the standard protocol.

Subsequently the gel was stored for 2 weeks within the release chambers at 37°C without the addition of any release buffer. For matters of comparison a solution of EPO (4.0 mg/g) was also incubation in the release chambers at 37°C. At predetermined points of time 0.05 g of the VPG and 20µl of the solution were removed and analysed by reverse phase chromatography. In the case of VPGs, EPO was extracted from the lipid matrix prior analysis.

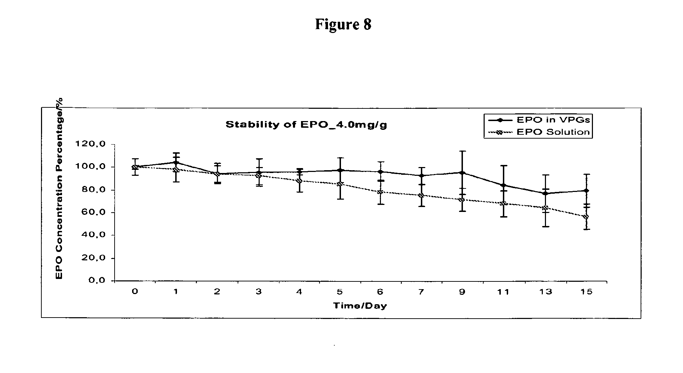

As it can be seen in Figure 8, the concentration of EPO within the solution and within the VPGs decreased over time. Beside, the monomer peak at all time points no degradation products were observed. This indicates that EPO was lost either due to aggregation and/or precipitation (highly aggregated EPO might not be assessable by reverse phase chromatography) or due to adsorption of EPO to the walls of the release chambers. Importantly, the loss of EPO was even more pronounced in solution which points to a stabilisation of EPO within the investigated VPG compositions.

## Claims

1. A pharmaceutical composition for sustained release of a pharmaceutically active compound, comprising a vesicular phospholipid gel having a viscosity of at least 3.0 Pa·s at 25°C at a shear rate of 38 s⁻¹ comprising tightly packed liposomes having a vesicular structure, wherein
(i) the vesicular phospholipid gel comprises at least one proteinaceous substance as the pharmaceutically active compound in encapsulated form, the at least one proteinaceous substance being a biologically active protein, peptide or polypeptide, wherein the at least one proteinaceous substance has a molecular weight of at least 3,400 g/mol; and
(ii) the gel exhibits a substantially continuous release rate of the at least one proteinaceous substance over a period of at least 200 hours when exposed at 37 °C to an aqueous medium.

2. The pharmaceutical composition according to claim 1, wherein the phospholipid is selected from the group consisting ofphosphatidylcholines, phosphatidylethanolamines, phosphatidylinosites, phosphatidylserines, cephalines, lysolecithines, phosphatidylglycerols and mixtures of several such phospholipids.

3. The pharmaceutical composition according to claim 1 or 2, wherein a blend of phosphatidylcholines and/or phosphatidylglycerols is used as the phospholipid.

4. The pharmaceutical composition according to any of claims 1 to 3, having a phospholipid content of at least 30 wt-%.

5. The pharmaceutical composition according to any of claims 1 to 4, further comprising a lipid selected from the group consisting of fatty acids, monoglycerides, diglycerides, triglycerides, sorbitan fatty acid esters, sphingolipids, cholesterol, waxes, and salts and derivatives thereof.

6. The pharmaceutical composition according to any of claims 1 to 5, wherein the weight ratio between the phospholipid and the at least one proteinaceous substance is between 1000:1 and 5:1.

7. The pharmaceutical composition according to any of claims 1 to 6, wherein the proteinaceous substance is an antibody selected from the group consisting of abciximab, adalimumab, alemtuzumab, basiliximab, bevacizumab, cetuximab, daclizumab, eculizumab, efalizumab, ibritumomab tiuxetan, infliximab, muromonab-CD3, natalizumab, omalizumab, palivizumab, panitumumab, ranibizumab, gemtuzumab ozogamicin, rituximab, tositumomab and trastuzumab.

8. The pharmaceutical composition according to any of claims 1 to 6, wherein the proteinaceous substance is a growth factor selected from the group consisting of epidermal growth factors, neuregulins, fibroblast growth factors, hematopoietic cytokines, insulin-like growth factors, interleukins, neurotrophic factors, platelet derived growth factors, transforming growth factors, tumor necrosis factors and VEGF.

9. The pharmaceutical composition according to any of claims 1 to 6, wherein the proteinaceous substance is a proteohormone selected from the group consisting of cytokines such as granulocyte-colony stimulating factor, interferons and interleukins; hematopoetic hormones such as erythropoietin; and peptide hormones such as follicle stimulating hormone, luteinizing hormone, gonadotropin-releasing hormone, glucagon and insulin.

10. The pharmaceutical composition according to any of claims 1 to 9, exhibiting a release of the at least one proteinaceous substance of less than 20% during 24 hrs when exposed at 37°C to an aqueous medium.

11. The pharmaceutical composition according to any of claims 1 to 10, further comprising at least one excipient which
(i) modifies the release of the at least one proteinaceous substance from the liposomes and/or
(ii) modifies the biodegradation of the vesicular phospholipid gel and/or
(iii) stabilizes the at least one proteinaceous substance and/or
(iv) modifies the solubility of the at least one proteinaceous substance.

12. The pharmaceutical composition according to claim 11, wherein the excipient is selected from the group consisting of hydrophilic polymers, sugars, polyols, surfactants, antioxidants, cyclodextrins, water miscible organic solvents and watersoluble salts.

13. A method for the production of a pharmaceutical composition as defined in any of claims 1 to 12, comprising
(i) providing a preparation comprising at least one phospholipid and at least one proteinaceous substance, wherein the at least one proteinaceous substance is a biologically active protein, peptide or polypeptide;
(ii) homogenizing the preparation of (i) in a dual asymmetric centrifuge.

14. The method according to claim 13, further comprising controlling and/or adjusting the temperature of the preparation during the centrifugation process.

15. The method according to claim 13 or 14, wherein homogenizing the preparation is performed in a dual asymmetric centrifuge without using any homogenization aid.

16. A pharmaceutical composition according to any of claims 1 to 12 for the use in immunotherapy, comprising administering to a subject in need thereof, a therapeutically effective amount of the pharmaceutical composition, wherein the immunotherapy comprises (i) treating malignant cell growth by stimulating the immune system or (ii) treating an autoimmune disease or preventing rejection of transplanted organs or cells by reducing the normal immune response.

17. A pharmaceutical composition according to any of claims 1 to 12 for the use in stimulating selective tissue regeneration in the treatment of tissue defects, comprising administering to a subject in need thereof, a therapeutically effective amount of the pharmaceutical composition.

18. The pharmaceutical composition for use according to claim 17, wherein the tissue is selected from the group consisting of joints, muscle tissue, connective tissue, tendons, organs and skin.

19. The pharmaceutical composition for use according to any of claims 16 to 18, wherein administering the pharmaceutical composition comprises injection, needlefree injection, vaginal application, nasal application, urethral application, topical application, peroral application or surgery.

## Patentansprüche

1. Pharmazeutische Zusammensetzung zur anhaltenden Freisetzung einer pharmazeutisch aktiven Substanz umfassend ein vesikuläres Phospholipid-Gel mit einer Viskosität von wenigstens 3,0 Pa·s bei 25°C bei einer Scherrate von 38 s⁻¹ umfassend eng gepackte Liposomen mit einer vesikulären Struktur, wobei
(i) das vesikuläre Phospholipid-Gel wenigstens eine proteinöse Substanz als die pharmazeutisch aktive Substanz in verkapselter Form umfasst, wobei die wenigstens eine proteinöse Substanz ein biologisch aktives Protein, Peptid oder Polypeptid ist, wobei die wenigstens eine proteinöse Substanz ein Molekulargewicht von wenigstens 3400 g/mol hat; und
(ii) das Gel im Wesentlichen eine stetige Freisetzungsrate der wenigstens einen proteinösen Substanz von einer Dauer von wenigstens 200 Stunden, wenn bei 37°C einem wässrigen Medium ausgesetzt, zeigt.

2. Pharmazeutische Zusammensetzung gemäß Anspruch 1, wobei das Phospholipid ausgewählt ist aus der Gruppe bestehend aus Phosphatidylcholinen, Phosphatidylethanolaminen, Phosphatidylinositen, Phosphatidylserinen, Kephalinen, Lysolecithinen (Lysophosphatidylcholinen), Phosphatidylglycerinen und Gemische verschiedener solcher Phospholipide.

3. Pharmazeutische Zusammensetzung gemäß Anspruch 1 oder 2, wobei ein Gemisch von Phosphatidylcholinen und/oder Phosphatidylglycerinen als das Phospholipid verwendet wird.

4. Pharmazeutische Zusammensetzung gemäß einem der Ansprüche 1 bis 3 mit einem Phospholipidgehalt von wenigstens 30 Gew.-%.

5. Pharmazeutische Zusammensetzung gemäß einem der Ansprüche 1 bis 4, weiterhin umfassend ein Lipid ausgewählt aus der Gruppe bestehend aus Fettsäuren, Monoglyceriden, Diglyceriden, Triglyceriden, Sorbitanfettsäureestem, Sphingolipiden, Cholesterol, Waxen und Salzen und Derivaten davon.

6. Pharmazeutische Zusammensetzung gemäß einem der Ansprüche 1 bis 5, wobei das Gewichtsverhältnis zwischen dem Phospholipid und der wenigstens einen proteinösen Substanz zwischen 1000:1 und 5:1 liegt.

7. Pharmazeutische Zusammensetzung gemäß einem der Ansprüche 1 bis 6, wobei die proteinöse Substanz ein Antikörper ist ausgewählt aus der Gruppe bestehend aus Abciximab, Adalimumab, Alemtuzumab, Basiliximab, Bevacizumab, Cetuximab, Daclizumab, Eculizumab, Efalizumab, Ibritumomab-Tiuxetan, Infliximab, Muromonab-CD3, Natalizumab, Omalizumab, Palivizumab, Panitumumab, Ranibizumab, Gemtuzumab-Ozogamicin, Rituximab, Tositumomab und Trastuzumab.

8. Pharmazeutische Zusammensetzung gemäß einem der Ansprüche 1 bis 6, wobei die proteinöse Substanz einen Wachstumsfaktor ist ausgewählt aus der Gruppe bestehend aus epidermalen Wachstumsfaktoren, Neuregulinen, Fibroblasten-Wachstumsfaktoren, hämatopoetischen Cytokinen, Insulinähnlichen Wachstumsfaktoren, Interleukinen, neurotrophen Faktoren, Thrombozytenabgeleiteten Wachstumsfaktoren, transformierenden Wachstumsfaktoren, Tumornekrosefaktoren und VEGF.

9. Pharmazeutische Zusammensetzung gemäß einem der Ansprüche 1 bis 6, wobei die proteinöse Substanz ein Proteohormon ist ausgewählt aus der Gruppe bestehend aus Cytokinen wie der Granulozyten-Kolonie stimulierende Faktor, Interferonen und Interleukinen, hämatopoetischen Hormonen wie Erythropoietin und Peptidhormonen wie das follikelstimulierende Hormon, luteinisierende Hormon, Gonadoliberin, Glucagon und Insulin.

10. Pharmazeutische Zusammensetzung gemäß einem der Ansprüche 1 bis 9, welche eine Freisetzung der wenigstens einen proteinösen Substanz von weniger als 20% während 24 Stunden, wenn bei 37°C einem wässrigen Medium ausgesetzt, aufweist.

11. Pharmazeutische Zusammensetzung gemäß einem der Ansprüche 1 bis 10, weiterhin umfassend wenigstens einen Hilfsstoff, welcher
(i) die Freisetzung der wenigstens einen proteinösen Substanz aus den Liposomen modifiziert und/oder
(ii) die Biodegradation des vesikulären Phospholipid-Gels modifiziert und/oder
(iii) die wenigstens eine proteinöse Substanz stabilisiert und/oder
(iv) die Löslichkeit der wenigstens einen proteinösen Substanz modifiziert.

12. Pharmazeutische Zusammensetzung gemäß Anspruch 11, wobei der Hilfsstoff ausgewählt ist aus der Gruppe bestehend aus hydrophilen Polymeren, Zuckern, Polyolen, grenzflächenaktiven Stoffen, Antioxidantien, Cyclodextrinen, wassermischbaren organischen Lösungsmitteln und wasserlöslichen Salzen.

13. Verfahren für die Herstellung einer pharmazeutischen Zusammensetzung wie in einem der Ansprüche 1 bis 12 definiert umfassend
(i) das Bereitstellen einer Zusammensetzung umfassend wenigstens ein Phospholipid und wenigstens eine proteinöse Substanz, wobei die wenigstens eine proteinöse Substanz ein biologisch aktives Protein, Peptid oder Polypeptid ist;
(ii) das Homogenisieren der Zusammensetzung von (i) in einer dualen asymmetrischen Zentrifuge.

14. Verfahren gemäß Anspruch 13, weiterhin umfassend das Kontrollieren und/oder Anpassen der Temperatur der Zusammensetzung während des Zentrifugationsprozesses.

15. Verfahren gemäß Anspruch 13 oder 14, wobei das Homogenisieren der Zusammensetzung in einer dualen asymmetrischen Zentrifuge ohne die Verwendung jeglicher Homogenisierungshilfen durchgeführt wird.

16. Pharmazeutische Zusammensetzung gemäß einem der Ansprüche 1 bis 12 zur Verwendung bei Immuntherapie umfassend das Verabreichen einer therapeutisch wirksamen Menge der pharmazeutischen Zusammensetzung an ein Subjekt, welches sie benötigt, wobei die Immuntherapie (i) das Behandeln von malignem Zellwachstum durch Stimulieren des Immunsystems oder (ii) das Behandeln einer Autoimmunerkrankung oder das Vorbeugen der Abstoßung von transplantierten Organen oder Zellen durch Verringerung der normalen Immunantwort umfasst.

17. Pharmazeutische Zusammensetzung gemäß einem der Ansprüche 1 bis 12 zur Verwendung zum Stimulieren selektiver Geweberegeneration bei der Behandlung von Gewebedefekten umfassend das Verabreichen einer therapeutisch wirksamen Menge der pharmazeutischen Zusammensetzung an ein Subjekt, welches sie benötigt.

18. Pharmazeutische Zusammensetzung zur Verwendung gemäß Anspruch 17, wobei das Gewebe ausgewählt ist aus der Gruppe bestehend aus Gelenken, Muskelgewebe, Bindegewebe, Sehnen, Organen und Haut.

19. Pharmazeutische Zusammensetzung zur Verwendung gemäß einem der Ansprüche 16 bis 18, wobei das Verabreichen der pharmazeutischen Zusammensetzung Injektion, nadelfreie Injektion, vaginale Applikation, nasale Applikation, urethrale Applikation, topische Applikation, perorale Applikation oder Operation umfasst.

## Revendications

1. Composition pharmaceutique pour la libération soutenue d'un composé pharmaceutiquement actif, comprenant un gel phospholipidique vésiculaire ayant une viscosité d'au moins 3,0 Pa·s à 25 °C à un taux de cisaillement de 38 s⁻¹ comprenant des liposomes agencés de manière serrée ayant une structure vésiculaire, dans laquelle
(i) le gel phospholipidique vésiculaire comprend au moins une substance protéique en tant que le composé pharmaceutiquement actif sous forme encapsulée, l'au moins une substance protéique étant une protéine, un peptide ou un polypeptide biologiquement active/actif, sachant que l'au moins une substance protéique a un poids moléculaire d'au moins 3,400 g/mol ; et
(ii) le gel présente un taux de libération sensiblement continu de l'au moins une substance protéique sur une période d'au moins 200 heures lorsqu'il est exposé à 37 °C à un fluide aqueux.

2. La composition pharmaceutique selon la revendication 1, dans laquelle le phospholipide est sélectionné dans le groupe constitué par les phosphatidylcholines, les phosphatidyléthanolamines, les phosphatidylinosites, les phosphatidylsérines, les céphalines, les lysolécithines, les phosphatidylglycérols et les mélanges de plusieurs de tels phospholipides.

3. La composition pharmaceutique selon la revendication 1 ou 2, dans laquelle un mélange de phosphatidylcholines et/ou de phosphatidylglycérols est utilisé en tant que le phospholipide.

4. La composition pharmaceutique selon l'une quelconque des revendications 1 à 3, ayant une teneur en phospholipide d'au moins 30 % en poids.

5. La composition pharmaceutique selon l'une quelconque des revendications 1 à 4, comprenant en outre un lipide sélectionné dans le groupe constitué par les acides gras, les monoglycérides, les diglycérides, les triglycérides, les esters d'acides gras de sorbitan, les sphingolipides, le cholestérol, les cires, et les sels et dérivés de ceux-ci.

6. La composition pharmaceutique selon l'une quelconque des revendications 1 à 5, dans laquelle le rapport de poids entre le phospholipide et l'au moins une substance protéique est compris entre 1000:1 1 et 5:1.

7. La composition pharmaceutique selon l'une quelconque des revendications 1 à 6, dans laquelle la substance protéique est un anticorps sélectionné dans le groupe constitué par l'abciximab, l'adalimumab, l'alemtuzumab, le basiliximab, le bevacizumab, le cetuximab, le daclizumab, l'eculizumab, l'efalizumab, l'ibritumomab tiuxetan, l'infliximab, le muromonab-CD3, le natalizumab, l'omalizumab, le palivizumab, le panitumumab, le ranibizumab, le gemtuzumab ozogamicin, le rituximab, le tositumomab et le trastuzumab.

8. La composition pharmaceutique selon l'une quelconque des revendications 1 à 6, dans laquelle la substance protéique est un facteur de croissance sélectionné dans le groupe constitué par les facteurs de croissance épidermiques, les neurégulines, les facteurs de croissance de fibroblastes, les cytokines hématopoïétiques, les facteurs de croissance analoques à l'insuline, les interleukines, les facteurs neurotrophiques, les facteurs de croissance dérivés des plaquettes, les facteurs de croissance transformants, les facteurs de nécrose tumorale, et les VEGF (facteurs de croissance endothéliaux vasculaires).

9. La composition pharmaceutique selon l'une quelconque des revendications 1 à 6, dans laquelle la substance protéique est une protéohormone sélectionnée dans le groupe constitué par les cytokines telles que le facteur de stimulation de colonies de granulocytes, les interférons et les interleukines ; les hormones hématopoïétiques telles que l'érythropoïétine ; et les hormones de peptide telles que l'hormone de stimulation folliculaire, l'hormone lutéinisante, l'hormone de libération des gonadotropines, le glucagon et l'insuline.

10. La composition pharmaceutique selon l'une quelconque des revendications 1 à 9, présentant une libération de l'au moins une substance protéique de moins de 20 % pendant 24 heures lorsqu'elle est exposée à 37 °C à un fluide aqueux.

11. La composition pharmaceutique selon l'une quelconque des revendications 1 à 10, comprenant en outre au moins un excipient qui
(i) modifie la libération de l'au moins une substance protéique à partir des liposomes et/ou
(ii) modifie la biodégradation du gel phospholipidique vésiculaire et/ou
(iii) stabilise l'au moins une substance protéique et/ou
(iv) modifie la solubilité de l'au moins une substance protéique.

12. La composition pharmaceutique selon la revendication 11, dans laquelle l'excipient est sélectionné dans le groupe constitué par les polymères hydrophiles, les sucres, les polyols, les surfactants, les antioxydants, les cyclodextrines, les solvants organiques miscibles dans l'eau et les sels solubles dans l'eau.

13. Procédé pour la production d'une composition pharmaceutique telle que définie dans l'une quelconque des revendications 1 à 12, comprenant
(i) la mise à disposition d'une préparation comprenant au moins un phospholipide et au moins une substance protéique, sachant que l'au moins une substance protéique est une protéine, un peptide ou un polypeptide biologiquement active/actif ;
(ii) l'homogénéisation de la préparation de (i) dans une centrifugeuse asymétrique duale.

14. Le procédé selon la revendication 13, comprenant en outre la commande et/ou le réglage de la température de la préparation pendant le processus de centrifugation.

15. Le procédé selon la revendication 13 ou 14, dans lequel l'homogénéisation de la préparation est effectuée dans une centrifugeuse asymétrique duale sans utilisation d'une quelconque aide à l'homogénéisation.

16. Composition pharmaceutique selon l'une quelconque des revendications 1 à 12, destinée à être utilisée en immunothérapie, comprenant l'administration à un sujet qui en a besoin, d'une quantité thérapeutiquement efficace de la composition pharmaceutique, sachant que l'immunothérapie comprend (i) le traitement de la croissance de cellules malignes par stimulation du système immunitaire ou (ii) le traitement d'une maladie auto-immune ou la prévention du rejet d'organes ou de cellules greffés par réduction de la réponse immunitaire normale.

17. Composition pharmaceutique selon l'une quelconque des revendications 1 à 12, destinée à être utilisée dans la stimulation de la régénération sélective de tissu dans le traitement de défauts tissulaires, comprenant l'administration à un sujet qui en a besoin, d'une quantité thérapeutiquement efficace de la composition pharmaceutique.

18. La composition pharmaceutique destinée à être utilisée selon la revendications 17, dans laquelle le tissu est sélectionné dans le groupe constitué par les articulations, le tissu musculaire, le tissu conjonctif, les tendons, les organes et la peau.

19. La composition pharmaceutique destinée à être utilisée selon l'une quelconque des revendications 16 à 18, dans laquelle l'administration de la composition pharmaceutique comprend l'injection, l'injection sans aiguille, l'application vaginale, l'application nasale, l'application urétrale, l'application topique, l'application per-orale ou la chirurgie.
